# EUROPEAN PATENT APPLICATION

(11) **EP 4 520 835 A1**
(43) Date of publication of application: **12.03.2025**
(21) Application number: 23196595.5
(22) Date of filing: 11.09.2023
(51) Int. Cl.: C12P 5/00, C12P 7/00, C12P 7/02, C12P 7/26, C12P 7/62

(54) **ENZYMATIC MONOCYCLIZATION OF TERPENES BY LYCOPENE CYCLASES**

(71) Applicant: Universität Stuttgart, 70174 Stuttgart (DE)
(72) Inventor: Schneider, Andreas, 70771 Leinfelden-Echterdingen (DE); Hauer, Bernhard, 67136 Fußgönnheim (DE); Horz, Philip, 70569 Stuttgart (DE); Schell, Kristina, 50733 Köln (DE); Aberle, Benjamin, 75392 Deckenpfronn (DE); Lystbaek, Thomas, 70563 Stuttgart (DE)
(74) Representative: Schrell, Andreas

(57) **Abstract**

The present invention relates to a process for preparing a composition comprising a compound of formula II 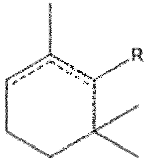 from a substrate of formula I 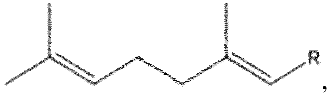 wherein R is an alkyl residue with at most 20 carbon atoms or an alkyl residue with at most 20 carbon atoms including at least one functional group, in particular wherein the functional group comprises at least one halide, O, N, S, P and/or unsaturated C atom, in at least one reaction medium and with at least one biocatalyst comprising a protein with the enzymatic activity of a small target(ST)-lycopene-β-cyclase (LcyB), which ST-LcyB comprises an amino acid sequence of a LcyB and is characterised by the presence of a first set of conserved amino acids F281, Y283, E295, T297, S298, E332, P337, G353, A356, G364 and M366 by reference to the wild type LcyB of SEQ ID No. 1.

## Description

The present invention relates to a process for preparing a composition comprising apocarotenoids, in particular vitamin A, dihydroionones, ionones and damascones, and a composition obtained thereby.

Monocyclic terpenes play fundamental roles as cell signaling or defense molecules in all known life forms. For example, vitamin A, as shown in scheme 1, below, is an essential molecule for an intact photoreceptor of the human retina, and abscisic acid, as shown in scheme 1, functions as a plant hormone that regulates the reaction to exterior stressors. Moreover, monocyclic terpenes such as dihydroionones, ionones or damascones, as shown in scheme 1, often find their application in the flavor and fragrance industry, due to their pleasant scents. As a result, the efficient generation of these broadly applicable molecules is of high economic interest. Naturally, these so called apocarotenoids are usually produced via oxidative cleavage of C40 carotenoids, which entails byproducts such as the mono- or dialdehydes, Cataldo et al., Appl Microbiol Biotechnol, 100, 2016, 5703-5718. Apocarotenoids are thus smaller molecules than carotenoids.

In nature, lycopene cyclases are the key enzymes that generate terminally monocyclic carotenoids from C40 terpene lycopene (as shown in scheme 1). Practical applications of these enzymes have been reported and include the preparation of carotenoids, Ma, Y. et al., Nat Commun, 13, 2022, 572, with different deprotonation patterns at the cyclohexane moiety or accumulation of lycopene by inhibition of the cyclase enzyme in metabolic pathways, Zhao et al., J. Agric. Food Chem., 68, 43, 2020, 11895-11907. Moreover, cyclization of a C30 lycopene-analog diaponeurosporene to diapotorulene was demonstrated, Lee, Pyung Cheon, et al. Chemistry & biology 10.5, 2003, 453-462.These enzymes are not used to prepare apocarotenoids with at most 29 carbon atoms, hereinafter also referred to smaller than C30 apocarotenoids.

In scheme 1, below, the following molecules are shown: vitamin A corresponds to 1, abscisic acid corresponds to 2, β-ionone corresponds to 3, α-ionone corresponds to 4, β-damascone corresponds to 5 and natural reaction of lycopene corresponding to 6 with a lycopene-β-cyclase which produces cyclic carotenoid precursors corresponding to 7.

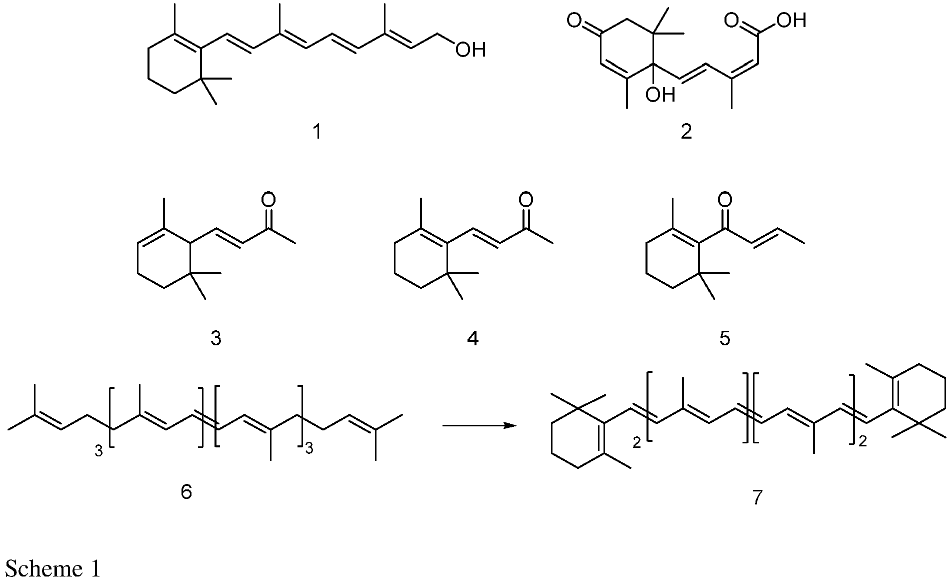

Chemically, monocyclic terpenes, in particular apocarotenoids, are produced via acid-catalyzed cyclization usually under cryogenic conditions, which depending on the substrate, entails polycyclic side-products, polymerization products and aborted cyclization products. Another common strategy is the scaffold remodeling approach which entails multi-step sequential chemistry starting from chiral pool molecules, Brill et al., Chem. Rev., 117, 18, 2017, 11753-11795. Finally, monocyclic terpenes, in particular apocarotenoids, are most commonly produced via unsustainable bottom-up chemistry starting from naphta-derived formaldehyde and isobutene, Parker, Tetrahedron, 72, 13, 2016, 1645-1652. As a result, a target-oriented monocyclization of linear precursors within a single step could drastically shorten synthetic routes.

Recently, in WO 2022 / 106522 the target-oriented monocyclization of C13-terpenes by an engineered squalene-hopene-cyclase was described. In this document, an active-site engineering approach is disclosed to overcome the priorly mentioned polycyclization of the substrate. The designed catalyst is highly specific for monocyclization of the C13 substrate.

Thus, there is an urgent need to provide means and methods for a simple approach not requiring active-site engineering or chemical synthesis routes in order to provide apocarotenoids, in particular with at most 29 carbon atoms.

The technical problem underlying the present invention therefore is to overcome aforementioned disadvantages, in particular to provide a simple, cost- and time effective and safe process to prepare apocarotenoids, in particular with at most 29 carbon atoms, and compositions obtained thereby, in particular to prepare vitamin A, dihydroionones, ionones or damascones.

The technical problem underlying the present invention is in particular solved by the teaching of the independent and dependent claims and the accompanying description.

The technical problem underlying the present invention is in particular solved by the provision of a process for preparing a composition comprising a compound of formula II 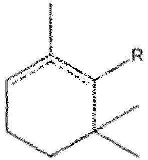 with R having the meaning of R in formula I, comprising the following steps:
a) providing a substrate of formula I 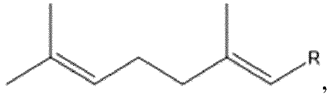 wherein R is an alkyl residue with at most 20 carbon atoms or an alkyl residue with at most 20 carbon atoms including at least one functional group, in particular wherein the functional group comprises at least one halide, O, N, S, P and/or unsaturated C atom,
   at least one reaction medium and at least one biocatalyst comprising a protein with the enzymatic activity of a ST (small target)-lycopene β-cyclase (LcyB) (hereinafter also referred to as ST-LcyB), which ST-LcyB comprises an amino acid sequence of a LcyB and is characterised by the presence of a first set of conserved amino acids F281, Y283, E295, T297, S298, E332, P337, G353, A356, G364 and M366 by reference to the wild type LcyB of SEQ ID No. 1,
b) mixing and reacting the substrate with the reaction medium and the biocatalyst provided in step a) and under reaction conditions so as to convert the substrate to the compound of formula II and
c) obtaining a composition comprising the compound of formula II.

The technical problem underlying the present invention is in particular also solved by the provision of a process for preparing a compound of formula II 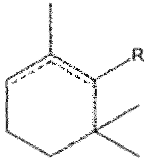 with R having the meaning of R in formula I, comprising the following steps:
a1) providing a substrate of formula I 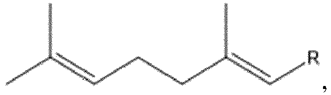 wherein R is an alkyl residue with at most 20 carbon atoms or an alkyl residue with at most 20 carbon atoms including at least one functional group, in particular wherein the functional group comprises at least one halide, O, N, S, P and/or unsaturated C atom,
   and at least one biocatalyst comprising a protein with the enzymatic activity of a ST (small target-lycopene β-cyclase (LcyB), which ST-LcyB comprises an amino acid sequence of a LcyB and is characterised by the presence of a first set of conserved amino acids F281, Y283, E295, T297, S298, E332, P337, G353, A356, G364 and M366 by reference to the wild type LcyB of SEQ ID No. 1,
b1) mixing and reacting the substrate with the biocatalyst provided in step a1) and under reaction conditions so as to convert the substrate to the compound of formula II and
c1) obtaining a composition comprising the compound of formula II.

Thus, according to the invention, in a first step a) or a1) a substrate of formula I 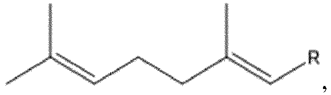 wherein R is an alkyl residue with at most 20 carbon atoms, in particular 2 to 20 carbon atoms, or wherein R is an alkyl residue with at most 20 carbon atoms, in particular 2 to 20 carbon atoms, including at least one functional group, in particular wherein the functional group comprises at least one halide, O, N, S, P and/or unsaturated C atom,
at least one reaction medium, preferably aqueous reaction medium, preferably at least an aqueous buffer, in step a) or being present optionally in step a1) and at least one biocatalyst is provided. Thus, the substrate may be an alkyl residue with or without at least one functional group. The functional group comprises or consists of a O, N, S, P or unsaturated C atom or any combination thereof.

Preferably, the substrate of formula I has at most 29 carbon atoms, in particular 10 to 29 carbon atoms.

In a second step b) or in step b1) the substrate of formula I is mixed and reacted with the biocatalyst under suitable reaction conditions, preferably in the reaction medium, so as to convert, in particular in a cyclization, in particular terminal monocyclization, reaction, the substrate to a compound of formula II 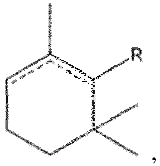 with R having the meaning of R in formula I,
which is obtained in the reaction mixture according to step c) or c1) and can subsequently be isolated therefrom. According to the invention, the R in formula II has the same meaning as R in formula I.

In a preferred embodiment the conversion in step b) or b1) of the substrate of formula I provided in step a) or a1) is a cyclization, in particular a monocyclization, in particular a terminal monocyclization, reaction. Preferably, the cyclization, in particular monocyclization, takes place at a terminal position of the substrate of formula I resulting in a terminal cyclization, in particular terminal monocyclization. Preferably, the conversion in step b) or b1) of the substrate of formula I is a monocyclization, in particular terminal monocyclization, resulting in a monocyclic, in particular terminal monocyclic, compound of formula II.

The invention enables to provide from a substrate, thus starting material, having the molecule structure of formula I, in a cyclization reaction a composition comprising a compound having the molecule structure of formula II, in particular it enables to produce a cyclic, in particular monocyclic, compound of formula II. These compounds represent compounds being to a large extent of great interest both from commercial and technological perspectives. The invention enables to provide in a single and straight-forward step a cyclization of a substrate of formula I, in particular a linear terpene, to a compound of formula II, in particular an apocarotenoid, in particular with at most 29 carbon atoms. For this purpose, the present invention employs a particular biocatalyst comprising a protein with the enzymatic activity of a ST small target-lycopene-β-cyclase (LcyB), which ST-LcyB comprises an amino acid sequence of a LcyB and is characterised by the presence of a first set of conserved amino acids F281, Y283, E295, T297, S298, E332, P337, G353, A356, G364 and M366 by reference to the wild type LcyB of SEQ ID No. 1, capable of converting a substrate of formula I in a cyclization reaction to a compound of formula II. Accordingly, the biocatalyst provided and used according to the invention is advantageously used for the cyclization, in particular monocyclization, in particular terminal monocyclization, of a substrate of formula I. According to the invention, therefore, a particularly simple, easily manageable and inexpensive cyclization reaction of a substrate of formula I is made possible, so that a high apparatus-related and cost-related effort as well as recombinant enzyme technology are avoided. The present invention is also advantageous in that it enables a single step cyclization, in particular monocyclization, reaction of small molecules, thus substrates of formula I which have at most 29 carbon atoms, and accordingly enables the preparing of cyclic, in particular monocyclic, compounds of formula II without the need for costly and extensive further steps.

According to the invention, the at least one biocatalyst comprises a protein with the enzymatic activity of a small target ST-lycopene-β-cyclase (LcyB).

A protein with the enzymatic activity of a ST-LcyB is in one preferred embodiment a ST-LcyB and thus comprises an amino acid sequence of a LcyB and is characterised by the presence of a first set of conserved amino acids, preferably also a second and preferably also a third set of conserved amino acids.

A protein with the enzymatic activity of a ST-LcyB is in another preferred embodiment a functional derivative of a ST-LcyB which has an amino acid sequence being different to that of the ST-LcyB but still having the enzymatic activity of a ST-LcyB.

A functional derivative of the ST-LcyB, in particular of a wild type LcyB of SEQ ID Nos 1, 2, 7 or 9, is further characterized by the presence of at least one specific amino acid replacement and/or the at least one specific difference in the order of the amino acids, in particular the presence of at least one additional amino acid and/or the presence of at least one deletion of at least one amino acid and/or comprises at least one amino acid inversion in the amino acid sequence of the ST-LcyB, in particular of a wild type LcyB of SEQ ID Nos 1, 2, 7 or 9, and thus is a mutated amino acid sequence. The mutated amino acid sequence of the ST-LcyB, in particular of an wild type LcyB of SEQ ID Nos 1, 2, 7 or 9, containing said at least one specific amino acid replacement and/or the at least one specific difference in the order of the amino acids, in particular the presence of at least one additional amino acid, the presence of at least one deletion of at least one amino acid and/or at least one amino acid inversion, is the amino acid sequence of the functional derivative and is termed herein to be a " mutated amino acid sequence".

The functional derivative comprising, in particular consisting of, the mutated amino acid sequence of the wild type LcyB of SEQ ID Nos 1, 2, 7 or 9 preferably has a specified degree of amino acid sequence identity to the wild type LcyB of SEQ ID Nos 1, 2, 7 or 9 or comprises at least a set of conserved amino acids as identified herein, or both of these features.

In a preferred embodiment, the functional derivative of the ST-LcyB, in particular of the wild type LcyB of SEQ ID Nos 1, 2, 7 or 9, has an amino acid sequence identity from 38,0 to 99,9 %, in particular 40,0 to 99,9 %, in particular 50,0 to 99,9 %, in particular 60,0 to 99,9 %, in particular 70,0 to 99,9 %, in particular 80,0 to 99,9 %, in particular 90,0 to 99,9 %, in particular 95,0 to 99,9 %, in particular 98,0 to 99,9 % to the amino acid sequence of the ST-LcyB.

The sequence identity is determined using the algorithm of Pearson and Litman (Proc. Natl. Acad. Sci USA, 85 (8), 1988, 2444 - 2448). The identity is an identity of the amino acids based on the entire length of the amino acid sequences.

In a preferred embodiment, the protein of the at least one biocatalyst comprising a protein with the enzymatic activity of a small target (ST)-lycopene-β-cyclase (LcyB) comprises, in particular consists of, an amino acid sequence with a length of at least 100, preferably at least 200, preferably at least 300, preferably at least 400, preferably at least 500, preferably at least 550, preferably at least 600 amino acids, preferably at least 700 amino acids, preferably at least 800 amino acids. Preferably, the sequence identity of the mutated amino acid sequence to the wild type LcyB of SEQ ID Nos 1, 2, 7 or 9 is given over the entire length of the mutated amino acid sequence.

In a particularly preferred embodiment, the functional derivative of the ST-LcyB, in particular of a wild type LcyB of SEQ ID Nos 1, 2, 7 or 9, comprises, in particular consists of, a mutated amino acid sequence with a length of at least 100, preferably at least 200, preferably at least 300, preferably at least 400, preferably at least 500, preferably at least 550, preferably at least 600 amino acids, preferably at least 700 amino acids, preferably at least 800 amino acids. Preferably, the sequence identity of the mutated amino acid sequence to the wild type LcyB of SEQ ID Nos 1, 2, 7 or 9 is given over the entire length of the mutated amino acid sequence.

In a particularly preferred embodiment, the functional derivative of the ST-LcyB, in particular of an wild type LcyB of SEQ ID Nos 1, 2, 7 or 9, comprises at least 1 to 11, preferably 3 to 11, preferably 5 to 11, preferably 7 to 11, preferably 9 to 11, preferably 11, amino acids of the first set of conserved amino acids F281, Y283, E295, T297, S298, E332, P337, G353, A356, G364 and M366, in particular at least amino acids S298 and M366, by reference to the wild type LcyB of SEQ ID No. 1.

In a furthermore preferred embodiment of the present invention, the functional derivative of the ST-LcyB, in particular of a wild type LcyB of SEQ ID Nos 1, 2, 7 or 9, has an amino acid sequence identity from 38,0 to 99,9 % to the amino acid sequence of the wild type LcyB of SEQ ID Nos 1, 2, 7 or 9, in particular 40,0 to 99,9 %, in particular 50,0 to 99,9 %, in particular 60,0 to 99,9 %, in particular 70,0 to 99,9 %, in particular 80,0 to 99,9 %, in particular 90,0 to 99,9 %, in particular 95,0 to 99,9 %, in particular 98,0 to 99,9 % and comprises at least 1 to 11, preferably 3 to 11, preferably 5 to 11, preferably 7 to 11, preferably 9 to 11, preferably 11, amino acids of the first set of conserved amino acids F281, Y283, E295, T297, S298, E332, P337, G353, A356, G364 and M366, in particular at least amino acids S298 and M366, by reference to the wild type LcyB of SEQ ID No. 1.

In a preferred embodiment of the present invention, a protein with the enzymatic activity of a ST-LcyB is a ST-LcyB comprising, in particular consisting of, an amino acid sequence of a LcyB , in particular an amino acid sequence of a wild type LcyB of SEQ ID Nos 1, 2, 7 or 9, and is characterised by the presence of a first set of conserved amino acids F281, Y283, E295, T297, S298, E332, P337, G353, A356, G364 and M366, preferably also a second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 and preferably also a third set of conserved amino acids W142, G219, Q236, G240, M258, D259, F281, Y283, E295, T297, G322, E332, P337, G353, A356, H360, P361, G364, A374, E408, F438 and L454, by reference to the wild type LcyB of SEQ ID No. 1 or is a functional derivative of the ST-LcyB having an amino acid sequence identity from 38,0 to 99,9 %, in particular 40,0 to 99,9 %, in particular 50,0 to 99,9 %, in particular 60,0 to 99,9 %, in particular 70,0 to 99,9 %, in particular 80,0 to 99,9 %, in particular 90,0 to 99,9 %, in particular 95,0 to 99,9 %, in particular 98,0 to 99,9 % to the amino acid sequence of the ST-LcyB.The ST-LcyB according to the present invention comprises, in particular consists of, an amino acid sequence of a LcyB and is characterised by the presence of a first set of conserved amino acids F281, Y283, E295, T297, S298, E332, P337, G353, A356, G364 and M366 by reference to the wild type LcyB of SEQ ID No. 1.

Thus, according to the invention the biocatalyst comprises a protein which is characterised by having the enzymatic activity of the ST-LcyB, which ST-LcyB is comprising, in particular consisting of, an amino acid sequence, which is identical to a wild-type amino acid sequence of a lycopene-β-cyclase from natural sources, for instance plant or microbial sources, and which ST-LcyB is further characterised by the presence of a first set of conserved amino acids F281, Y283, E295, T297, S298, E332, P337, G353, A356, G364 and M366 by reference to the wild type LcyB of SEQ ID No. 1 (hereinafter also termed "first set of conserved amino acids"). The biocatalyst enables to convert a substrate of formula I with at most 29 carbon atoms, which is thus a small target, to a compound of formula II under reaction conditions so as to convert the substrate of formula I to the compound of formula II. Thus, the present invention enables to convert in a reaction medium various substrate of formula I, which have at most 29 carbon atoms to compounds of formula II by using at least one biocatalyst comprising a protein with the enzymatic activity of a small target (ST)-lycopene-β-cyclase (LcyB) comprising an amino acid sequence of a LcyB characterized by the presence of the first set of conserved amino acids.

In the context of the present invention, the identification of the position of a conserved amino acid in an amino acid sequence of a protein with the enzymatic activity of a lycopene-β-cyclase (LcyB), in particular ST-LcyB, is preferably done by aligning the amino acid sequence of the LcyB to the amino acid sequence of the wild type LcyB of SEQ ID No. 1, in particular aligning their sets of conserved amino acids.

Preferably, the amino acid sequence of the wild type LcyB of SEQ ID No. 1 comprises a set of conserved amino acids, which set of conserved amino acids can be found in identical or corresponding positions in at least one other amino acid sequence of a wild type LcyB selected from the group consisting of SEQ ID No. 2, 7 and 9, preferably all of them.

The biocatalyst provided in step a) or a1) of the process of the present invention is characterized by its enzymatic activity, in particular by its ability to enzymatically catalyse, in particular in a liquid reaction medium, at least the cyclization of a substrate of formula I to a compound of formula II.

According to the invention, the residue R of the substrate of formula I is an alkyl residue or an alkyl residue including at least one functional group, in particular wherein the functional group comprises at least one halide, O, N, S, P and/or unsaturated C atom, each with at most 20 carbon atoms, in particular 2 to 20 carbon atoms. In a preferred embodiment, the alkyl residue or the alkyl residue with at most 20 carbon atoms including at least one functional group, in particular wherein the functional group comprises at least one halide, O, N, S, P and/or unsaturated C atom, of the substrate of formula I has at most 19, in particular at most 15, in particular at most 10, in particular at most 7 in particular at most 5, in particular at most 3, in particular at most 2, in particular 2 to 20, in particular 2 to 15, in particular 2 to 10 , in particular 2 to 7, in particular 2 to 5, in particular 16 to 20, in particular 10 to 20, in particular 5 to 20, in particular 10 to 20, in particular 10 to 15, carbon atoms.

Thus, according to the invention the alkyl residue or the alkyl residue with at most 20 carbon atoms including at least one functional group, in particular wherein the functional group comprises at least one halide, O, N, S, P and/or unsaturated C atom, of the compound of formula II has, due to the present invention's reaction being a cyclisation, the same number of carbon atoms, that means at most 20 carbon atoms, in particular 2 to 20 carbon atoms. In a preferred embodiment, the alkyl residue or the alkyl residue with at most 20 carbon atoms including at least one functional group, in particular wherein the functional group comprises at least one halide, O, N, S, P and/or unsaturated C atom, of the compound of formula II has at most 19, in particular at most 15, in particular at most 10, in particular at most 7 in particular at most 5, in particular at most 3, in particular at most 2, in particular 2 to 20, in particular 2 to 15, in particular 2 to 10, in particular 2 to 7, in particular 2 to 5, in particular 16 to 20, in particular 10 to 20, in particular 5 to 20, in particular 10 to 20, in particular 10 to 15, carbon atoms.

According to the invention, the substrate of formula I has at most 29 carbon atoms. In a preferred embodiment, the substrate of formula I has at most 28, in particular at most 26, in particular at most 24, in particular at most 20, in particular at most 15, in particular at most 11, in particular at most 10, in particular 1 to 29, in particular 1 to 28, in particular 1 to 25, in particular 1 to 20, in particular 10 to 29, in particular 10 to 25, in particular 15 to 29, in particular 15 to 25, in particular 20 to 29, in particular 20 to 25, carbon atoms.

Thus, according to the invention the compound of formula II has, due to the present invention's reaction being a cyclisation, the same number of carbon atoms, that means at most 29 carbon atoms. In a preferred embodiment, the compound of formula II has at most 28, in particular at most 26, in particular at most 24, in particular at most 20, in particular at most 15, in particular at most 11, in particular at most 10, in particular 1 to 29, in particular 1 to 28, in particular 1 to 25, in particular 1 to 20, in particular 10 to 29, in particular 10 to 25, in particular 15 to 29, in particular 15 to 25, in particular 20 to 29, in particular 20 to 25, carbon atoms.

According to the invention, R in formula I and II can be an alkyl residue having at most 20 carbon atoms, in particular from 2 to 20 carbon atoms. According to the invention, R in formula I and II can be an alkyl residue having at most 20 carbon atoms, in particular from 2 to 20 carbon atoms including at least one functional group, in particular wherein the functional group comprises at least one halide, O, N, S, P and/or unsaturated C atom. According to the invention, the compound of formula I and II has at most 29, in particular at most 28, in particular at most 26, in particular at most 24, in particular at most 20, in particular at most 15, in particular at most 11, in particular at most 10, in particular 1 to 29, in particular 1 to 28, in particular 1 to 25, in particular 1 to 20, in particular 10 to 29, in particular 10 to 25, in particular 15 to 29, in particular 15 to 25, in particular 20 to 29, in particular 20 to 25, carbon atoms including the carbon atoms of R in formula I and 11, wherein R is an alkyl residue having at most 20 carbon atoms, in particular from 2 to 20 carbon atoms or an alkyl residue with at most 20 carbon atoms including at least one functional group, in particular wherein the functional group comprises at least one halide, O, N, S, P and/or unsaturated C atom, including optionally present C atoms in the functional group.

In a preferred embodiment, the functional group of the alkyl residue is selected from a group selected from the group consisting of a fluoride group, a chloride group, a bromide group, an iodide group, a hydroxy group, a ketone group, an aldehyde group, a halo formyl group, a carbonate ester group, a carboxylate group, a carboxyl group, a carboalkoxy group, an ether group, an acetal group, a carboxylic anhydride group, a carboxamide group, an amidine group, an amine group, a ketimine group, an imide group, an azide group, a diimide group, a cyanate group, an isocyanate group, a nitrate group, a nitrile group, a isonitrile group, a nitrite group, a nitro group, a nitroso group, a oxime group, a pyridyl group, a carbamate group, a sulfhydryl group, a sulfide group, a disulfide group, a sulfinyl group, a sulfonyl group, a sulfino group, a sulfo group, a thiocyanate group, a isothiocyanate group, a carbonothioyl group, a carbothioic S-acid group, a carbothioic O-acid group, a thioester group, a thionoester group, a carbodithioic acid group, a carbodithio group, a phosphino group, a phosphono group, a phosphate group, an alkenyl group, in particular an unsaturated methylene group, an alkynyl group, in particular a methine group, and a combination thereof.

In a preferred embodiment, the at least one functional group is selected from the group consisting of a hydroxy, a ketone, a carbonate ester, an oxybenzyl group and a combination thereof.

In a preferred embodiment, R in formula I and formula II is selected from a group consisting of CH₃, 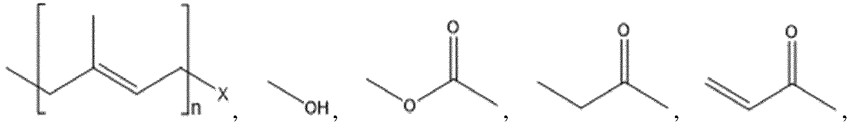 and 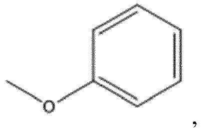 wherein X is selected from a group consisting of H, OH, 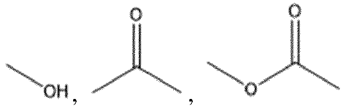 and 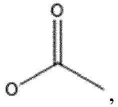 and
wherein n is 1 to 3.

In a preferred embodiment, the substrate is selected from the group consisting of a substrate having the substrate number 8, in particular 8 9, 10, 11, in particular 11 12, 13 ,14, 15, 16, 17 and 18 in scheme 2 in the example 3 of the present description.

In a preferred embodiment, the substrate of formula I is an acyclic precursor of vitamin A, dihydroionone, an ionone, a damascone or a derivative thereof.

In a preferred embodiment, the compound of formula II is represented by formula IIa or lib, with R having the meaning of R in formula I,

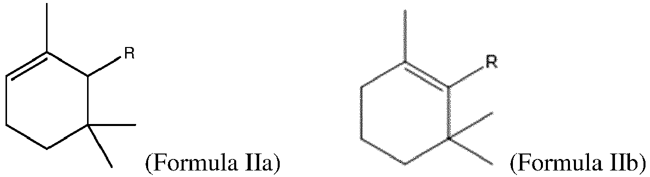

In a preferred embodiment, the compound of formula II is vitamin A, dihydroionone, β-ionone, α-ionone, β-damascone, α-damascone or a derivative thereof.

In a preferred embodiment, the composition comprising the compound of formula II obtained in step c) or c1) comprises at least 0.1 mol%, in particular 0.2 mol%, in particular 1.0 mol%, in particular 1.2 mol%, in particular 2.5 mol%, in particular 3.0 mol%, in particular 5.0 mol%, in particular 6.0 mol%, in particular 10.0 mol%, in particular 30.0 mol%, in particular 50.0 mol%, in particular 62.0 mol%, in particular 75.0 mol%, in particular 0.1 to 100 mol%, in particular 0.2 to 90 mol%, in particular 1.0 to 80 mol%, in particular 1.2 to 70 mol%, in particular 2.5 to 65 mol%, in particular 5.0 to 62 mol% of the compound of formula II in relation to the total molecular amount of the composition comprising the compound of formula II.

In a preferred embodiment, the composition comprising the compound of formula II obtained in step c) or c1) comprises at least 0.1 wt.-%, in particular 0.2 wt.-%, in particular 1.0 wt.-%, in particular 1.2 wt.-%, in particular 2.5 wt.-%, in particular 3.0 wt.-%, in particular 5.0 wt.-%, in particular 6.0 wt.-%, in particular 10.0 wt.-%, in particular 30.0 wt.-%, in particular 50.0 wt.-%, in particular 62.0 wt.-%, in particular 75.0 wt.-%, in particular 0.1 to 100 wt.-%, in particular 0.2 to 90 wt.-%, in particular 1.0 to 80 wt.-%, in particular 1.2 to 70 wt.-%, in particular 2.5 to 65 wt.-%, in particular 5.0 to 62 wt.-% of the compound of formula II in relation to the total weight of the composition comprising the compound of formula II (dry weight to total weight).

In a preferred embodiment, the reaction of the substrate of formula I to the compound of formula II in step c) or step c1) takes place with a conversion of at least 0.1 %, in particular 0.2 %, in particular 1.0 %, in particular 1.2 %, in particular 2.5 %, in particular 3.0 %, in particular 5.0 %, in particular 6.0 %, in particular 10.0 %, in particular 30.0 %, in particular 50.0 %, in particular 62.0 %, in particular 75.0 %, in particular 0.1 to 100 %, in particular 0.2 to 90 %, in particular 1.0 to 80 %, in particular 1.2 to 70 %, in particular 2.5 to 65 %, in particular 5.0 to 62 %, of the substrate of formula I to the compound of formula II, in relation to the molecular amount of the substrate of formula I.

In a preferred embodiment, the biocatalyst is used in the reaction medium in step b) or b1) in a quantity that in the reaction medium a concentration from 0,001 to 100 µmol/l, preferably 0,01 to 90 µmol/l, preferably 0,1 to 80 µmol/l, preferably 1 to 75 µmol/l, preferably 10 to 70 µmol/l, preferably in particular 40 to 60 µmοl/l of the protein with the enzymatic activity of a ST-LcyB, in relation to the reaction medium is present.

In a preferred embodiment, the biocatalyst is used in the reaction medium in step b) or b1) in a quantity that in the reaction medium a concentration from 0,001 to 100 µmol/l, preferably 0,01 to 90 µmol/l, preferably 0,1 to 80 µmol/l, preferably 1 to 75 µmol/l, preferably 10 to 70 µmol/l, preferably in particular 40 to 60 µmol/l of the protein with the enzymatic activity of a ST-LcyB, in relation to the amount of water or buffer in the reaction medium is present.

In a preferred embodiment, the biocatalyst, in particular host cell, is used in the reaction medium in step b) or b1) at a cell concentration of 0,1 to 100 mg/ml, in particular 10 to 80 mg/ml, in particular 30 to 60 mg/ml, in particular 50 mg/ml, in relation to the reaction medium (wet weight to total volume of reaction medium).

In a preferred embodiment, the biocatalyst, in particular host cell, is used in the reaction medium in step b) or b1) at a cell concentration of 0,1 to 100 mg/ml, in particular 10 to 80 mg/ml, in particular 30 to 60 mg/ml, in particular 50 mg/ml, in relation to the volume of water or buffer in the reaction medium (wet weight to total volume of water or buffer).

In a particularly preferred embodiment, the substrate of formula I is used in step b) or b1) in the reaction medium in a concentration from 0.1 to 10 mmol/l, preferably 0.5 to 5 mmol/l, in particular from 0.7 to 3 mmol/l, preferably 1 mmol/l, in relation to the reaction medium.

In a particularly preferred embodiment, the substrate of formula I is used in step b) or b1) in the reaction medium in a concentration from 0.1 to 10 mmol/l, preferably 0.5 to 5 mmol/l, in particular from 0.7 to 3 mmol/l, preferably 1 mmol/l, in relation to the amount of water or buffer in the reaction medium.

In a particularly preferred embodiment, the reaction medium in step b) or optionally step b1) is water, at least one buffer, in particular at least one aqueous buffer or a buffer system of two of them.

In a particularly preferred embodiment, the reaction medium in step b) or optionally step b1) comprises water, preferably is water, preferably demineralized water, preferably distilled water.

In a preferred embodiment, the reaction medium in step b) or optionally step b1) comprises, preferably consists of, a buffer, in particular an aqueous buffer, in particular selected from the group of buffering agents consisting of phosphate buffer, in particular potassium phosphate buffer, in particular 10× phosphate buffer, a potassium buffer, in particular MES/potassium buffer, in particular MES/KOH buffer, TRIS-Maleate buffer, citrate buffer and acetate buffer, in particular citrate buffer.

In a preferred embodiment, the reaction medium comprises, in particular consists of, a citrate buffer.

Preferably, the buffer comprises 50 to 500, preferably 90 to 200, preferably 100 mM of the buffering agent in water.

Most preferably, the citrate buffer comprises 50 to 500, preferably 90 to 200, preferably 100 mM citric acid in water.

Most preferably, the citrate buffer comprises 50 to 500, preferably 90 to 200, preferably 100 mM citric acid at a pH of 5,5 to 6,5, preferably 5,8 to 6,2, preferably 6,0 in water.

In a preferred embodiment, the reaction medium comprises, in particular consists of, at least two different buffers, in particular aqueous buffers, thus, a buffering system.

In a preferred embodiment, the reaction medium comprises a buffer system comprising, in particular consisting of, at least one buffer, in particular selected from the group consisting of phosphate buffer, in particular potassium phosphate buffer, in particular 10× phosphate buffer, a potassium buffer, in particular MES/potassium buffer, in particular MES/KOH buffer, TRIS-Maleate buffer, citrate buffer and acetate buffer, in particular citrate buffer, and water, preferably demineralized water, preferably distilled water.

In a preferred embodiment, the reaction medium in step y) has a pH of 3,0 to 8,0, in particular 4,0 to 7,5, in particular 5,0 to 7,0, in particular 5,5 to 6,5, in particular the reaction medium in process step y) has a pH of 4,0, in particular 5,0, in particular 5,5, in particular 6,0, in particular 6,5, in particular 7,0, in particular 8,0.

In a preferred embodiment, the reaction medium comprises at least one co-factor, in particular NADPH or FMN, in particular NADPH and FMN.

In a preferred embodiment, the reaction temperature in step b) or b1) is 20 °C to 50 °C, in particular 22 °C to 42 °C, in particular 25 °C to 40 °C, in particular 28 °C to 32 °C, in particular 30 °C.

In a particularly preferred embodiment, the reaction in step b) or b1) takes place without cryogenic conditions.

In a preferred embodiment, the pH of the reaction medium is from 5 to 10, in particular 3,0 to 8,0, in particular 4,0 to 7,5, in particular 5,0 to 7,0, in particular 5,5 to 6,5, in particular 6.

In a preferred embodiment, the reaction time of the reaction in step b) or b1) is 1 to 50 h, in particular 10 to 30 h, in particular 20 h.

In a preferred embodiment of the present invention, step b) or b1) is carried out under mechanical agitation, in particular shaking or stirring.

In a preferred embodiment of the present invention, step b) or b1) is carried out under mechanical agitation, in particular shaking or stirring, preferably with 1 to 5000 rpm, preferably 10 to 3000 rpm, preferably 100 to 800 rpm, preferably 200 to 800 rpm.

In a preferred embodiment the reaction in step b) or b1) is stopped in a step b2) by adding an organic solvent, in particular ethyl acetate and/or cyclohexane, in particular ethyl acetate and cyclohexane in a 1 to 1 (v/v) mixture.

In a preferred embodiment, the biocatalyst provided in step a) or a1) of the process according to the present invention comprises, in particular consists of, an isolated protein, a protein mixture, or a protein-comprising system, which system is a host cell, a host cell lysate, a freeze-dried host cell, a host cell extract or a host cell fraction and wherein the protein has the enzymatic activity of a ST-lycopene β-cyclase (LcyB).

In a preferred embodiment, the biocatalyst provided in step a) or a1) of the process of the present invention is a host cell comprising the protein with the enzymatic activity of a ST-LcyB. Preferably, the protein with the enzymatic activity of a ST-LcyB is expressed in the host cell. Preferably, the protein with the enzymatic activity of a ST-LcyB is physically associated with the host cell, in particular intracellularly located or membrane bound, and shows a biocatalytic performance, thus, a conversion according to step b) of the substrate of formula I provided in step a) to the compound of formula II. Preferably, the biocatalyst used in the process of the present invention is an isolated protein with the enzymatic activity of a ST-LcyB or a protein with the enzymatic activity of a ST-LcyB which is physically associated with the host cell, in particular intracellularly located or membrane bound, in which it was expressed.

Preferably, the protein with the enzymatic activity of a ST-LcyB remains physically associated with a host cell, in particular intracellularly located in the host cell which expressed the protein with the enzymatic activity of a ST-LcyB and is obtained together with the host cell, preferably as a viable host cell or a non-viable host cell.

Preferably, the host cell containing the expressed protein with the enzymatic activity of a ST-LcyB and/or the host cell with which the expressed protein with the enzymatic activity of a ST-LcyB is physically associated, in particular intracellularly located or membrane bound, is further processed so as to produce host cell lysates, host cell extracts, freeze-dried host cell or cell fractions, in particular cell membrane fragments, cytoplasmic fractions or both, preferably all of them containing the expressed protein with the enzymatic activity of a ST-LcyB and/or with the host cell which the expressed protein with the enzymatic activity of a ST-LcyB is physically associated, in particular intracellularly located or membrane bound, and, therefore, can be used as the biocatalyst provided in step a) or a1) of the process according to the present invention.

In a particularly preferred embodiment, the biocatalyst provided in step a) or a1) in the process according to the present invention is a host cell, wherein the host cell may be a eukaryotic or prokaryotic host cell. In a preferred embodiment, the host cell is a microorganism, fungal cell, plant cell, animal cell or human cell. In a particularly preferred embodiment, the prokaryotic host cell may be a bacterial host cell, preferably E. coli. In a particularly preferred embodiment, the eukaryotic host cell may be an animal cell, in particular mammalian or insect cell, plant cell or fungal cell.

In a preferred embodiment, the biocatalyst comprises, in particular consists of, the cell membrane fragments of the host cell.

In a furthermore preferred embodiment, the host cell containing the protein with the enzymatic activity of a ST-LcyB, in particular in its cell membrane, is a viable cell, in particular whole cell.

In a further preferred embodiment, the host cell containing the protein with the enzymatic activity of a ST-LcyB is an inactivated or a dead cell, in particular whole cell.

In a furthermore preferred embodiment of the present invention, the biocatalyst containing protein with the enzymatic activity of a ST-LcyB is a host cell extract, freeze-dried host cell, a host cell lysate or a host cell fraction, in particular cell membrane fragments.

In a preferred embodiment, the biocatalyst may be provided in step a) of the process of the present invention in form of a powder or in liquid or semi-liquid form, for instance in form of a suspension.

In a preferred embodiment, the biocatalyst is a carrier-supported biocatalyst.

In a preferred embodiment, the biocatalyst, in particular host cell, comprising a protein with the enzymatic activity of a ST-LcyB provided in step a) or a1) in the process according to the present invention is recombinantly produced, naturally occurring or is provided synthetically.

In a preferred embodiment, the ST-LcyB is characterised by the presence of a second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 by reference to the wild type LcyB of SEQ ID No. 1.

In a preferred embodiment, the ST-LcyB is characterised by the presence of a third set of conserved amino acids W142, G219, Q236, G240, M258, D259, F281, Y283, E295, T297, G322, E332, P337, G353, A356, H360, P361, G364, A374, E408, F438 and L454 by reference to the wild type LcyB of SEQ ID No. 1.

In a preferred embodiment, the ST-LcyB comprises, in particular consist of, an amino acid sequence of a wild type LcyB of SEQ ID Nos 1, 2, 7 or 9.

In a particularly preferred embodiment, the functional derivative of the ST-LcyB, in particular of an wild type LcyB of SEQ ID Nos 1, 2, 7 or 9, comprises at least 1 to 93, preferably 10 to 93, preferably 30 to 93, preferably 50 to 93, preferably 80 to 93, preferably 93, amino acids of the second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 by reference to the wild type LcyB of SEQ ID No. 1.

In a furthermore preferred embodiment of the present invention, the functional derivative of the ST-LcyB, in particular of an wild type LcyB of SEQ ID Nos 1, 2, 7 or 9, has an amino acid sequence identity from 38,0 to 99,9 % to the amino acid sequence of the wild type LcyB of SEQ ID Nos 1, 2, 7 or 9, in particular 40,0 to 99,9 %, in particular 50,0 to 99,9 %, in particular 60,0 to 99,9 %, in particular 70,0 to 99,9 %, in particular 80,0 to 99,9 %, in particular 90,0 to 99,9 %, in particular 95,0 to 99,9 %, in particular 98,0 to 99,9 % and comprises at least 1 to 93, preferably 10 to 93, preferably 30 to 93, preferably 50 to 93, preferably 80 to 93, preferably 93, amino acids of the second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 by reference to the wild type LcyB of SEQ ID No. 1.

In a preferred embodiment, the composition comprising, in particular consisting of, the compound of formula II in step c) or c1) is made available from the respective reaction medium or solvent, in particular is isolated from it in step c) or c1). In particular obtaining the composition in step c) or c1) is a concentrating or filtering off, in particular isolating, the desired composition, in particular from the respective reaction medium. Preferably the processes used for this can be physical, chemical processes or biological processes.

In a preferred embodiment, in step d) the compound of formula II is isolated from the composition obtained in step c) or c1).

In a preferred embodiment, the compound of formula II is isolated from the composition obtained in step c) or c1) by at least one filtration, extraction, in particular with at least one organic solvent, in particular with ethyl acetate and/or cyclohexane, distillation, chromatography or a combination thereof.

The present invention also provides a composition obtainable according to the process of the present invention.

In a preferred embodiment, the composition obtainable according to the process of the present invention comprises, in particular consists of a compound of formula II.

In a preferred embodiment, the composition obtainable according to the process of the present invention comprises, in particular consists of a compound of formula II, wherein the compound of formula II is a compound of formula IIa or lib, in particular is vitamin A, dihydroionone, β-ionone, α-ionone, β-damascone or α-damascone or a derivative thereof.

In the context of the present invention, a "protein with the enzymatic activity of a small target(ST)-lycopene-β-cyclase (LcyB)" is a lycopene beta(β)-cyclase and, thus, is a protein which is able to enzymatically catalyse the conversion, in particular cyclization, in particular monocyclization, in particular terminal monocyclization, of an acyclic terpene with at most 29 carbon atoms, in particular at most 28, in particular at most 26, in particular at most 24, in particular at most 20, in particular at most 15, in particular at most 11, in particular at most 10, in particular 1 to 29, in particular 1 to 28, in particular 1 to 25, in particular 1 to 20, in particular 10 to 29, in particular 10 to 25, in particular 15 to 29, in particular 15 to 25, in particular 20 to 29, in particular 20 to 25, carbon atoms, in particular a substrate of formula I, into a cyclic terpene, in particular a monocyclic terpene, in particular a terminal monocyclic terpene, in particular an apocarotenoid. Preferably, the protein with the enzymatic activity of a small target (ST)-lycopene-β-cyclase (LcyB) is able to catalyse said conversion having the same activity as LcyB_C according to SEQ ID No. 1 in aqueous citrate buffer, preferably in a 100 mM citrate buffer, preferably at 30 °C, preferably under the conditions such as exemplified in the examples, in particular example 3 and/or preferably with at least the conversion rates given in example 3 for the substrates tested.

A protein with the enzymatic activity of a ST-LcyB is in one preferred embodiment a ST-LcyB and thus comprises an amino acid sequence of a LcyB and is characterised by the presence of a first set of conserved amino acids, preferably also a second and preferably also a third set of conserved amino acids.

A protein with the enzymatic activity of a ST-LcyB is in another preferred embodiment a functional derivative of a ST-LcyB still having the enzymatic activity of a ST-LcyB, which is the ability to enzymatically catalyse the conversion, in particular cyclization, in particular monocyclization, in particular terminal monocyclization, of an acyclic terpene with at most 29 carbon atoms, in particular at most 28, in particular at most 26, in particular at most 24, in particular at most 20, in particular at most 15, in particular at most 11, in particular at most 10, in particular 1 to 29, in particular 1 to 28, in particular 1 to 25, in particular 1 to 20, in particular 10 to 29, in particular 10 to 25, in particular 15 to 29, in particular 15 to 25, in particular 20 to 29, in particular 20 to 25, carbon atoms, in particular a substrate of formula I, into a cyclic terpene, in particular a monocyclic terpene, in particular a terminal monocyclic terpene, in particular an apocarotenoid, preferably having the same activity as LcyB_C according to SEQ ID No. 1 in aqueous citrate buffer, preferably in a 100 mM citrate buffer, preferably at 30 °C, preferably under the conditions such as exemplified in the examples, in particular example 3 and/or preferably with at least the conversion rates given in example 3 for the substrates tested.

The specific ability of the "protein with the enzymatic activity of a small target (ST)-lycopene-β-cyclase (LcyB)", namely to act on small substrates having below 30 carbon atoms, does not mean, that the protein is not able to also catalyse a monocyclization of larger substrates. Thus, a "protein with the enzymatic activity of a small target (ST)-lycopene-β-cyclase (LcyB)" is able or is not able to cyclize, in particular monocyclize, substrates having 30 or more carbon atoms, but in any case, is able to cyclize, in particular monocyclize substrates with at most 29 carbon atoms, that means less than 30 carbon atoms. Preferably, the (ST)-LcyB has the enzymatic activity of a LcyB, in particular is a LcyB.

Preferably, the protein with the enzymatic activity of a ST-LcyB is classified as EC 5.5.1.19. In a preferred embodiment, it is a prokaryotic enzyme, particularly belonging to the terpene cyclase/mutase family. In particular, in the examples it is also termed enzyme.

In the context of the present invention, a "small target (ST)" is understood to mean a small target molecule, in particular organic molecule, which is converted by a protein with the enzymatic activity of a ST-LcyB. The small target molecule has at most 29 carbon atoms.

In the context of the present invention, the term "lycopene beta(β)-cyclase" (LcyB) refers to an enzyme with the systematic name carotenoid beta-end group lyase (decyclizing), which enzyme catalyses the following chemical reaction: carotenoid ψ-end group ⇆ carotenoid β-end group.
LcyB has the EC classification EC 5.5.1.19.

Preferably, this enzyme requires NAD(P)H. It converts the acyclic carotenoid lycopene (2 ψ ends) into the cyclic β-carotene (2 β ends).

Lycopene cyclases generate provitamin A carotenoids which are essential building blocks for cyclic xanthophylls involved in photosynthesis and regulatory networks. The cyclization of lycopene is the final step in carotenoid biosynthesis and may proceed via one of two pathways: the formation of a β-ring by beta-cyclase, or an ε-ring by epsilon-cyclase. Epsilon-cyclase adds only one ring, forming the monocyclic δ-carotene, whereas beta-cyclase can introduce a ring at both ends of lycopene to form the bicyclic β-carotene.

Lycopene cyclases are widely distributed across taxonomic groups and have structural diversity. Four partly related families of lycopene cyclases are known: CrtY, CrtL (β-ionone end group producing), CrtL (η-ionone end group producing) and CrtL (capsanthin/capsorubin synthase).

In the context of the present invention, the term "apocarotenoids" refers to cleavage products from carotenoids, that means products which are obtainable by cleavage, in particular oxidative cleavage of carotenoids, which also includes products which are not produced by cleavage or oxidative cleavage from carotenoids but which have the same chemical formula and structure, in particular vitamin A, dihydroionones, ionones and damascones

In the context of the present invention, the term "formula II 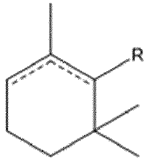 with R having the meaning of R in formula I," means "formula II 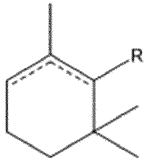 wherein R is an alkyl residue with at most 20 carbon atoms or an alkyl residue with at most 20 carbon atoms including at least one functional group, in particular wherein the functional group comprises at least one halide, O, N, S, P and/or unsaturated C atom."

In the context of the present invention, "physically associated with" is understood to mean, that a protein remains in a cell, in particular a host cell which expressed the protein, in particular the protein is intracellularly located or membrane bound.

In the context of the present invention "intracellularly located" is understood to mean, that the protein is located within a cell, in particular a host cell which expressed the protein.

In the context of the present invention "membrane bound" is understood to mean, that the protein is located in the cell membrane of a cell, in particular a host cell which expressed the protein.

In the context of the present invention, a conserved amino acid means an amino acid of an amino acid sequence of one species which is identical to an amino acid in one or more amino acid sequences of at least one other species in the corresponding position of the at least one other amino acid sequence, which corresponding position preferably is determined by sequence alignment, herein also termed aligning.

In the context of the present invention, a set of conserved amino acids is composed of at least 2 conserved amino acids which may be consecutive or non-consecutive, that means interrupted by an amino acid sequence gap, and which at least 2 amino acids are identical to at least 2 amino acids in the corresponding positions of at least one other amino acid sequence being aligned.

In the context of the present invention, the sequence alignment, is a way of arranging the sequences of amino acids to identify regions of identity. Aligned sequences of amino acids are represented as rows within a matrix, in particular and preferably the matrix presented in table 5 and 6. Gaps are, if necessary, inserted between the identical amino acids so that identical amino acids are aligned in successive columns. In a preferred embodiment of the present invention, the sequence alignment is done using the software Clustal Omega. This online tool is a bioinformatics sequence analysis application, which is free to use.

In the context of the present invention, the first or second set of conserved amino acids of the wild type LcyB (SEQ ID No 1) is identified by aligning the amino acid sequence of the wild type LcyB (SEQ ID No 1) with at least one other amino acid sequence of a wild type Lcy, preferably at least 2 or 3 other amino acid sequences of a wild type Lcy, preferably selected from the group consisting of SEQ ID Nos 2, 7 and 9, in particular using Clustal Omega.

In the context of the present invention, the term "a set of conserved amino acids at positions X by reference to the wild type LcyB of SEQ ID No. 1" means that the specific amino acids at positions X as referred to can be found at the indicated positions in the amino acid sequence of LcyB (SEQ ID No. 1), and at the same or a corresponding position in aligned amino acids sequences of a different wild type Lcy.

In the context of the present invention, a "functional derivative of an amino acid sequence of a protein" is a variant of the protein which comprises a mutated amino acid sequence comprising the amino acid sequence of the protein with the exception of the presence of at least one amino acid difference to the amino acid sequence of the protein, in particular at least one specific amino acid replacement and/or at least one specific difference in the order of the amino acids and/or the presence of at least one additional amino acid and/or the presence of at least one deletion of at least one amino acid and/or comprises at least one amino acid inversion in the amino acid sequence. Said functional derivative still retains the biological, in particular enzymatic activity, that means function of the protein.

In the context of the present invention, "compound" is understood to mean a molecule or several identical molecules.

In the context of the present invention, "alkyl residue" is understood to mean a linear or branched alkyl residue. Preferably, all carbon atoms of the alkyl residue are saturated carbon atoms, thus, only forming single bonds.

In the context of the present invention, "unsaturated carbon atom" is understood to mean a carbon atom which forms at least one double or triple bond.

In the context of the present invention, a "functional group" can be a substituent, atom or moiety in a molecule, preferably in a hydrocarbon, except a hydrogen or a saturated carbon atom, for example a substituent comprising at least one halide, O, N, S, P and/or unsaturated C atom. Preferably, the functional group is for example selected from a group consisting of a fluoride group, a chloride group, a bromide group, an iodide group, a hydroxy group, a ketone group, an aldehyde group, a halo formyl group, a carbonate ester group, a carboxylate group, a carboxyl group, a carboalkoxy group, an ether group, an acetal group, a carboxylic anhydride group, a carboxamide group, an amidine group, an amine group, a ketimine group, an imide group, an azide group, a diimide group, a cyanate group, an isocyanate group, a nitrate group, a nitrile group, a isonitrile group, a nitrite group, a nitro group, a nitroso group, a oxime group, a pyridyl group, a carbamate group, a sulfhydryl group, a sulfide group, a disulfide group, a sulfinyl group, a sulfonyl group, a sulfino group, a sulfo group, a thiocyanate group, a isothiocyanate group, a carbonothioyl group, a carbothioic S-acid group, a carbothioic O-acid group, a thioester group, a thionoester group, a carbodithioic acid group, a carbodithio group, a phosphino group, a phosphono group, a phosphate group, an alkenyl group, in particular an unsaturated methylene group, an alkynyl group, in particular methine group and a combination thereof. Preferably, a functional group is understood to mean any organic functional group, preferably for example -F, -Cl, -Br, -I, -OH, - NH₂, -SH, -PO₃H₂, or HC=CH-.

In the context of the present invention, the structural formula of vitamin A is

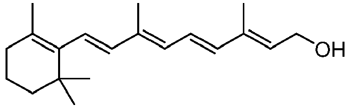

In the context of the present invention, the IUPAC (International Union of Pure and Applied Chemistry) name of vitamin A is (2E,4E,6E,8E)-3,7-dimethyl-9-(2,6,6-trimethylcyclohex-1-en-1-yl)nona-2,4,6,8-tetraen-1-ol.

In the context of the present invention, the CAS (Chemical Abstracts Service) number of vitamin A is 68-26-8.

In the context of the present invention, the structural formula of dihydroionone is

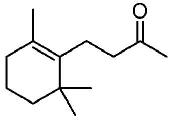

In the context of the present invention, the IUPAC (International Union of Pure and Applied Chemistry) name of dihydroionone is 4-(2,6,6-trimethylcyclohex-1-en-1-yl)butan-2-one.

In the context of the present invention, the CAS (Chemical Abstracts Service) number of dihydroionone is 17283-81-7.

In the context of the present invention, the structural formula of β-ionone is

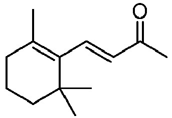

In the context of the present invention, the IUPAC (International Union of Pure and Applied Chemistry) name of β-ionone is (E)-4-(2,6,6-trimethylcyclohex-1-en-1-yl)but-3-en-2-one.

In the context of the present invention, the CAS (Chemical Abstracts Service) number of β-ionone is 14901-07-6.

In the context of the present invention, the structural formula of α-ionone is

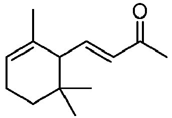

In the context of the present invention, the IUPAC (International Union of Pure and Applied Chemistry) name of α-ionone is (E)-4-(2,6,6-trimethylcyclohex-2-en-1-yl)but-3-en-2-one.

In the context of the present invention, the CAS (Chemical Abstracts Service) number of α-ionone is 127-41-3.

In the context of the present invention, the structural formula of β-damascone is

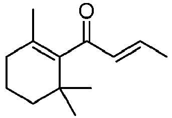

In the context of the present invention, the IUPAC (International Union of Pure and Applied Chemistry) name of β-damascone is (E)-1-(2,6,6-trimethylcyclohex-1-en-1-yl)but-2-en-1-one.

In the context of the present invention, the CAS (Chemical Abstracts Service) number of β-damascone is 35044-68-9.

In the context of the present invention, the structural formula of α-damascone is

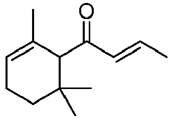

In the context of the present invention, the IUPAC (International Union of Pure and Applied Chemistry) name of α-damascone is (E)-1-(2,6,6-trimethylcyclohex-2-en-1-yl)but-2-en-1-one.

In the context of the present invention, the CAS (Chemical Abstracts Service) number of α-damascone is 43052-87-5.

In the context of the present invention, a "composition comprising a compound of formula II" is understood to mean a composition comprising a compound of formula II and optionally side products or the provided substrate of formula I. Preferably, the composition comprising a compound of formula II does not comprise any reaction medium but is obtained in step c) or c1) in the reaction medium.

In the context of the present invention the amino acids are presented in the one-letter-code, wherein A is alanine, R is arginine, N is asparagine, D is aspartic acid, C is cysteine, E is glutamic acid, Q is glutamine, G is glycine, H is histidine, I is isoleucine, L is leucine, K is lysine, M is methionine, F is phenylalanine, P is proline, S is serine, T is threonine, W is tryptophan, Y is tyrosine and V is valine.

In the context of the present invention the amino acids given in the first, second and third set of conserved amino acids are understood to be cumulatively present.

In the context of the present invention, the term "obtaining a composition" is understood to mean that the composition produced in a preceding step by reacting reactants is made available from the respective culture or reaction medium or solvent, in particular is isolated from it, in particular obtaining a composition is therefore to be understood preferably as concentrating or filtering off, in particular isolating, the desired composition. The processes used for this can be physical, chemical processes or biological processes.

In the context of the present invention, the term "and/or" is understood to mean that all members of a group connected by the term "and/or" are represented both cumulatively with respect to each other in any combination, and alternatively with respect to each other. Exemplarily, for the expression "A, B and/or C", the following disclosure is to be understood thereunder: i) (A or B or C), or ii) (A and B), or iii) (A and C), or iv) (B and C), or v) (A and B and C), or vi) (A and B or C), or vii) (A or B and C), or viii) (A and C or B).

In the context of the present invention, individual components or constituents of a composition or of one of its components, determined quantitatively in relative form, in particular in percentages, preferably add up to 100% by weight or molar amount of the respective composition referred to or of the composition or, if referred to, of a component thereof, unless otherwise stated.

Further preferred embodiments of the present invention are apparent from the dependent claims.

The present invention will be explained in more detail in the following examples and the accompanying figures both of which are not to be understood as limiting.

The sequence listing shows:
SEQ ID No. 1 represents the amino acid sequence of the wild type Lcy from Capsicum annuum (wild type LcyB_C or LcyB_Can). SEQ ID No. 2 represents the amino acid sequence of wild type Lcy of Arabidopsis thaliana (wild type LcyB_Ath). SEQ ID No. 3 represents the amino acid sequence of wild type Lcy of Arabidopsis thaliana (LcyE-Ath). SEQ ID No. 4 represents the amino acid sequence of wild type Lcy of Zea mays (LcyE-Zma) SEQ ID No. 5 represents the amino acid sequence of wild type Lcy of Dunaliella salina strain (LcyE-Dsa). SEQ ID No. 6 represents the amino acid sequence of wild type Lcya of Pantoea ananas (CrtY_Pan). SEQ ID No. 7 represents the amino acid sequence of wild type Lcy of Synechococcus elongatus (Crtl_syn). SEQ ID No. 8 represents the amino acid sequence of wild type Lcy of Camellia sinensis var. assamica (LcyE-Csi). SEQ ID No. 9 represents the amino acid sequence of wild type Lcy of Solanum lycopersicum (Tomato_B).

The figures show:
figure 1: two exemplary SDS-PAGEs show the lycopene-β-cyclases (lcys) of SEQ ID 1-9 in the range of 55 kDa expressed in E. coli;
figure 2: an overview of three GC chromatograms including a GC chromatogram which shows the production of β-ionone from pseudoionone using the LcyB_CAN of SEQ ID No 1 compared with the GC chromatograms of molecule standards and the empty vector control reaction;
figure 3: a diagram of a buffer evaluation using acetate = acetate buffer pH = 6.0; citrate = citrate buffer pH = 6.0; MES = 2-Morpholinoethanesulfonic acid pH = 6.0; Tris-Maleate = Tris(hydroxymethyl)aminomethane maleate pH = 7.5;
figure 4: a diagram of the conversion rates of pseudoionone to β-ionone using Leys according to SEQ ID Nos 1 to 9; and
figure 5: a) to k) respective GC chromatograms including the respective mass spectra as inlet of compounds of formula II from their respective substrates of formula I with substrate numbers 8E (E-isomer of 8), 9, 10, 11 12, 13, 14, 15, 16, 17 and 18.

### Examples:

**Table 1: List of buffers:**

| **Buffer** | **Ingredients** |
|---|---|
| 10x phosphate buffer (KPᵢ-buffer) | 0,17 M KH₂PO₄, 0,72 M K₂HPO₄, pH=7,4 |
| MES/KOH buffer | 100 mM MES, pH=6,0 |
| TRIS-Maleate buffer | 100 mM TRIS, pH=6,0 |
| Citrate buffer | 100 mM Citric acid, pH=6,0 |
| Acetate buffer | 100 mM Acetic acid, pH=6,0 |

| | |
|---|---|
| MES:2-(*N*-morpholino)ethanesulfonic acidTRIS: Tris-(hydroxymethyl)aminomethane | |

**Table 2: List of media:**

| **Medium** | **Ingredients** |
|---|---|
| Lysogeny broth | 10 g/L tryptone, 10 g/L NaCl, 5 g/L yeast extract |
| Terrific broth medium | 12 g/L tryptone, 24 g/L yeast extract, 4 g/L Glycerol, |

**Table 3: Composition of the PCR mixture:**

| **substance** | **volume [µl]** | **final concentration** |
|---|---|---|
| *dd*H₂O | 29 | |
| DMSO | 2,5 | |
| KOD Hot Start Buffer (10x) | 5 | 1x |
| dNTPs (2 mM each) | 5 | 250 µm (each) |
| MgSO₄ (25 mM) | 4,5 | 2 mM |
| Template DNA | 1 | 0,5-5 ng/ µl |
| Primer *forward* (10 µM) | 1 | 0,2 µm |
| Primer *reverse* (10 µM) | 1 | 0,2 µm |
| KOD Hot Start DNA Polymerase | 1 | |

**Table 4: PCR temperature profile:**

| **step** | **Temperature [°C]** | **time [s]** | **cycles** |
|---|---|---|---|
| Initial denaturation | 95 | 120 | 1 |
| Denature | 95 | 30 | |
| Annealing | 60 | 30 | 30 |
| Extension | 70 | 210 | |
| Final extension | 72 | 420 | 1 |

### Material:

- Chemicals: The chemicals used for syntheses, molecular biology and biochemical work have been purchased from Carl-Roth (Karlsruhe, DE), VWR (Pennsylvania, US), Sigma-Aldrich/ Merck (St. Louis, US) and Alfa-Aesar (Ward Hill, US).
- Molecular biological kits: The molecular biological kits for DNA-purification (Zymoclean DNA Clean & Concentrator Kit), Agarose gel-extraction (Zymoclean Gel DNA Recovery Kit) and plasmid isolation (Zyppy^{™}Plasmid Miniprep Kit) were purchased from ZymoResearch (Irvine, US).

Analytics:
- Nuclear Magnetic Resonance (NMR): ¹H- und ¹³C-NMR spectra were recorded on a Bruker Avance 500 Spectrometer at 500,15 MHz for ¹H- and 125 MHz for ¹³C. The chemical shifts δ are referred to tetramethylsilane (=TMS) in ppm set to 0. All substances were dissolved in CDCl₃ and recorded at room temperature.
- Gas chromatography (GC): GC analyses were performed using an Agilent GC 7820A equipped with a mass spectrometer MSD 5977B and a ZB1-HT capillary column (Phenomenex, 30 m × 250 µm × 0,25 µm) and helium as carrier gas with a constant pressure of 14.168 ψ. Injections (1 µl) were performed in split mode (10:1). GC conversion rates were calculated directly from GC-MS spectra by integration-quotient of substrates and products.

### Example 1: Preparation and analysis of lycopene cyclases

### Enzyme identification

A sequence similarity network based on the Lycopene-β- cyclase (lcys) from Capsicum anuum, hereinafter also referred to as lcyB_CAN, lcyB_Can, Can-LcyB or lcyB_C, represented by SEQ ID No 1 and known as Uniprot: Q43415, was generated with the Basic Local Alignment Search Tool (BLAST).

The first enzymes (LcyB-Can, LcyB-Ath, LcyE-Ath) were identified based on their natural reaction by literature research. The remaining sequences were selected due to homology from a pool of sequences generated with the Basic Local Alignment Search Tool (BLAST) using the three previously mentioned sequences as query. The sequences were analyzed as a sequence similarity network and by multi sequence alignment to obtain a diverse set of sequences representing a large range of the natural sequence space.

Based on that, nine lcys represented by SEQ ID Nos 1 to 9 for recombinant production in E. coli were selected.

### Gene synthesis and plasmid preparation

The DNA sequences for the lycopene cyclases were cloned in pDHE1650 plasmids using Gibson Assembly. The gene inserts were optimized for expression in *E. coli* and purchased as synthetic DNA (Twist Bioscience, USA). Vector backbone was obtained as linear DNA fragment by PCR.

Isolation of the plasmid proceeded following to the standard protocol of *Zyppy*^{™}*Plasmid Miniprep Kit* by ZymoResearch (Zymo Research. Zyppy^{™} Plasmid Miniprep Kit. Instr. Man. 4037, 1-9, 2014). For the photometric determination of the plasmid DNA concentration, 1 µL was measured on a Nanodrop 1000 (Agilent, Santa Clara, US) at a wavelength of 260 nm.

### Plasmid transformation via heat-shock method

Chemically competent *E. coli* TG 20+ cells based on rubidium chloride were produced for the transformation of the plasmid DNA. The transformation was carried out under sterile conditions. 1 µl of the purified (crude) PCR product was added to 25 µl TG 20+ competent cells and incubated for 30 min on ice, followed by a heat shock at 42 °C for 105 s with subsequent ice cooling for 3 min. After adding 500 µl of LB medium, the cells were incubated for 40 min at 37 °C, 100 µL were plated on petri dishes (Ampicillin, c_{end}= 100 µg/ml) and grown colonies were picked and grown in 5 ml LB medium (Ampicillin, c_{end}= 100 µg/ml). After isolation of the plasmid, sequencing for quality control was performed.

### 1.3: Expression of Ley in 2L flasks

Expression cultures were inoculated with 5 mL of the LB pre-culture into 500 mL of TB medium (Ampicillin, c_{end}= 100 µg/ml) with 0.5 g/L rhamnose as the inductor. The cultures were incubated for 20 h at 30 °C, 180 rpm and harvested afterwards (8000 rpm, 30 min). The resulting pellets were directly used for biotransformations, frozen at -20 °C overnight, or lyophilized after storage at -80 °C.

### Lyophilization protocol

Freshly harvested *E. coli* cells including the Lcy genes were transferred to petri dishes and frozen at -80 °C overnight. On the following day the frozen pellets were quickly transferred to a lyophilizer and lyophilized at -80°C at 0,0001 atm for two days. The resulting lyophilized whole cells including Lcy genes were mortared gently and stored in 50 ml Falcon tubes at room temperature.

### SDS-PAGE

After cell harvesting 50 mg of the cells were resuspended in 1 ml of *dd*H₂O, 15 µl of the resulting suspension were mixed with 5 µl SDS loading buffer and heated to 95 °C for 10 min. Afterwards 10 µl of the preparation was loaded on the pre-prepared SDS-PAGE (Expedeon).

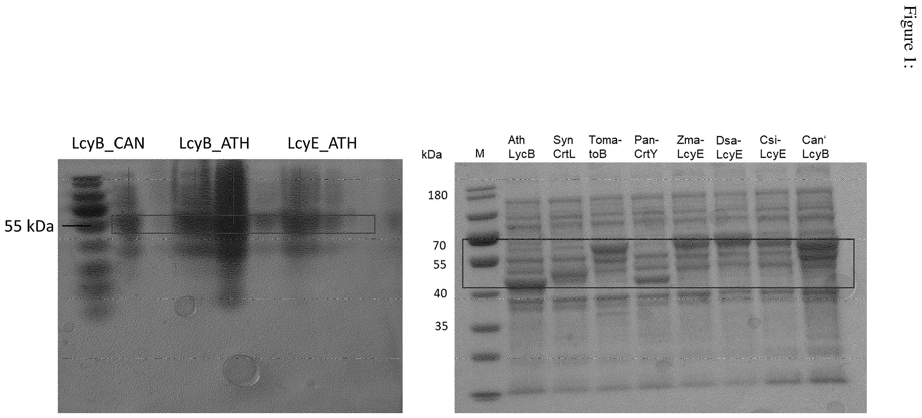

All selected lcys were successfully produced by the E. coli host, which was confirmed via the exemplary SDS-PAGEs in Figure 1. The left SDS-PAGE shows from left to right the LcyB-CAN (SEQ ID No 1), the LcyB-Ath (SEQ ID No 2) and LcyE-Ath (SEQ ID No 3) preparation and the right SDS-PAGE shows from left to right M, LcyB-Ath (SEQ ID No 2), Crtl-syn (SEQ ID No 7), Tomato-B (SEQ ID No 9), CrtY-Pan (SEQ ID No 6), LcyE-Zma (SEQ ID No. 4), LcyE-Dsa (SEQ ID No 5), LcyE-Csi (SEQ ID No 8) and LcyB-Can (SEQ ID No 1).

### Example 2: Engineering of a reaction of a lycopene substrate to an apocarotenoid product and analyzing the reaction conditions

### Choosing the engineering setup

The lcyB_CAN (SEQ ID No 1) was chosen for setup engineering. The C13 terpene 5E-pseudoionone was chosen as the lycopene substrate analog. As devised by the literature for substrates having at least 40 carbon atoms NADPH and FMN were used as cofactors and the reaction was carried out at 30 °C and 50 °C in TRIS-maleate buffer.

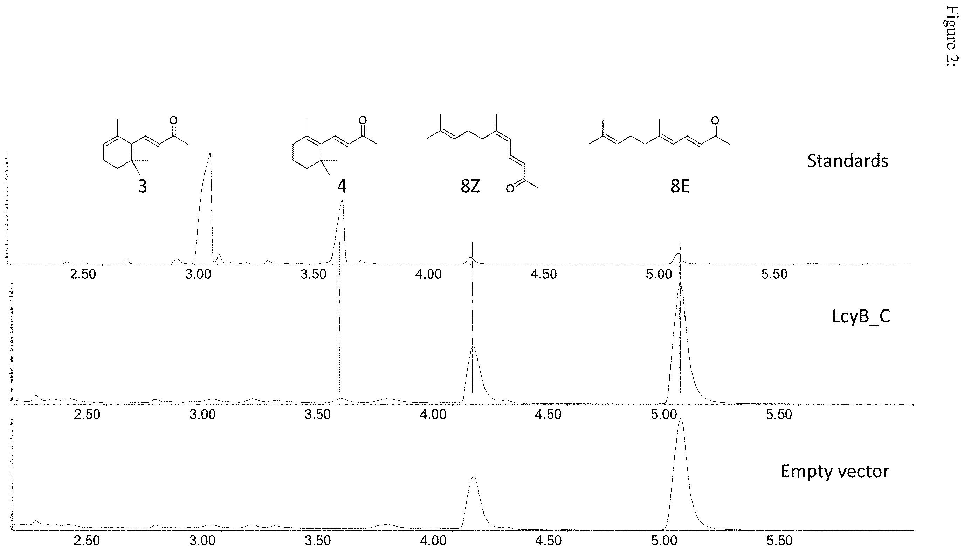

Surprisingly, it was found, that the lcyB_CAN (SEQ ID No 1) cyclized the substrate pseudoionone to β-ionone at 30 °C, which was confirmed with a product standard as shown in the GC chromatograms of Figure 2, wherein E-pseudoionon corresponds to 8E and Z-pseudoionon corresponds to 8Z, β-ionone corresponds to 4 and α-ionone corresponds to 3.

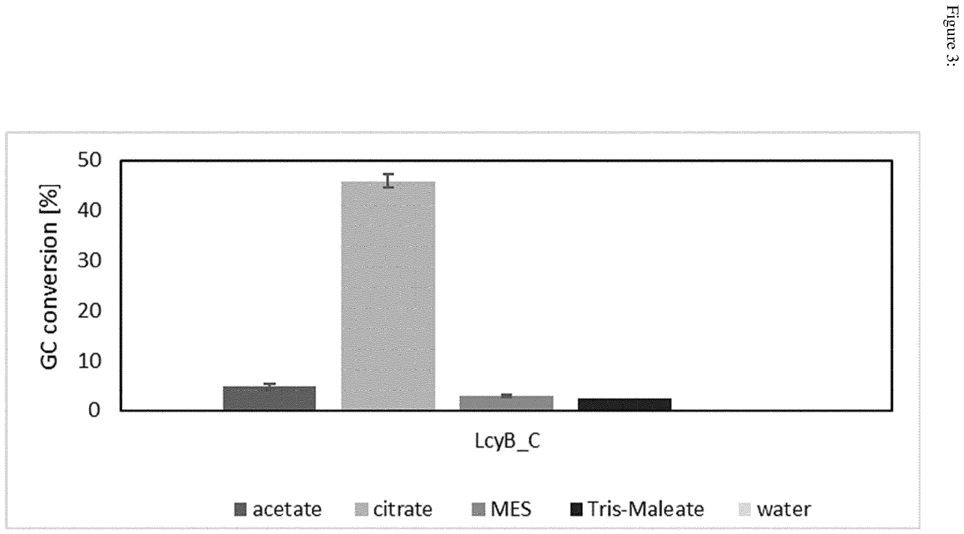

### Evaluating the buffers (Figure 3)

Next, the reaction was carried out using different buffers or water as reaction media at 30°C to evaluate the best buffer for the reaction. After enzyme production in E. coli, 50 mg cells as at least one biocatalyst were provided according to step a) of the process of the present invention and were resuspended in 1 ml buffer or water as at least one reaction medium also provided according to step a) of the process of the present invention, 0.02 mM FMN, 1 mM NAPDH and 1 mM substrate as substrate of formula I were provided according to step a) or a1) of the process of the present invention and added and the reaction mixture was stirred at 30 °C for 20 h according to step b) or b1) of the process of the present invention.

A 10-fold increase in activity using citric acid buffer could be detected and shown in Figure 3. Figure 3 shows a bar diagram with different bufferson the x-axis, from left to right: acetate, citrate, MES, Tris-Maleate and water. The GC conversion of the cyclization reaction of substrate pseudoionone to corresponding β-ionone using lcyB_CAN of SEQ ID No 1 as biocatalyst in the different buffersor water as reaction media is shown on the y-axis in % in relation to the molecular amount of substrate pseudoionone. Accordingly, while the best conversion rates could be shown in the citrate buffer significant conversions were also detected in the acetate, MES and Tris-Maleate buffers.

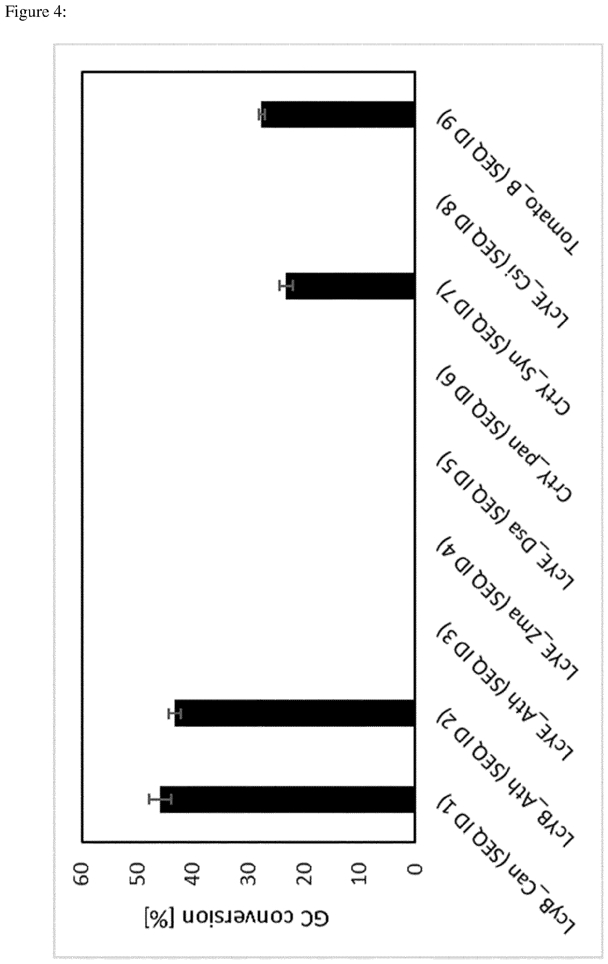

### Biotransformations and analysis of different Leys (Figure 4)

With essentially the setup as identified for the experiments of figure 3, the selected lycopene-β-cyclases were evaluated for their promiscuous pseudoionone cyclization ability.

After enzyme production in E. coli, 50 mg cells as at least one biocatalyst were provided according to step a) of the process of the present invention and were resuspended in 1 ml citrate buffer as at least one reaction medium also provided according to step a) of the process of the present invention, 0.02 mMFMN, 1 mM NAPDH and 1 mM substrate as substrate of formula I were provided according to step a) or a1) of the process of the present invention and added and the reaction mixture was stirred at 30 °C for 20 h according to step b) or b1) of the process of the present invention.

Reactions were stopped by adding ethyl acetate/cyclohexane (1:1) and inversion as well as vortexing of the suspension. After two extractions, the resulting organic phase, including substrates and products, thus a composition comprising the compound of formula II, which were obtained according to step c) or c1) of the present invention, was measured directly over GC-MS. Quantification was made directly by integration-quotient of substrates and products.

It was surprisingly found that next to wild type LcyB_C (SEQ ID 1), wild types LcyB_Ath (SEQ ID 2), CrtY_Syn (SEQ ID 7) and Tomato_B (SEQ ID 9) were also able to cyclize substrate pseudoionone to corresponding β-ionone with a conversion of 43.2 %, 23.2 % and 27.6 % in relation to the substrate pseudoionone, respectively, as shown in Figure 4. Figure 4 shows a bar diagram of the Lcys of SEQ ID No 1 to 9 on the x-axis from left to right starting from SEQ ID No 1 ending with SEQ ID No 9. The GC conversion of the cyclization reaction of substrate pseudoionone to corresponding β-ionone using the respective Leys as biocatalyst is shown on the y-axis in % in relation to the molecular amount of substrate pseudoionone. Figure 4 shows, that surprisingly biocatalysts comprising a protein with the enzymatic activity of a small target(ST)-lycopene-β-cyclase (LcyB), which ST-LcyB comprises an amino acid sequence of a LcyB and is characterised by the presence of a first set of conserved amino acids F281, Y283, E295, T297, S298, E332, P337, G353, A356, G364, M366 and a second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 by reference to the wild type LcyB of SEQ ID No. 1, are able to convert in a process according to the present invention a substrate of formula I to a compound of formula II.

### Example 3: Reactions of different linear terpene substrates to respective apocarotenoid products using LcyB_C as biocatalyst

Next, LcyB_C (SEQ ID 1) was taken with the buffer and a series of linear terpene substrates was tested. After enzyme production in E. coli, 50 mg cells of LcyB_C (SEQ ID 1) as at least one biocatalyst were provided according to step a) of the process of the present invention and were resuspended in 1 ml citrate buffer as at least one reaction medium also provided according to step a) of the process of the present invention, 0.02 mM FMN, 1 mM NAPDH and 1 mM substrate as substrate of formula I were provided according to step a) or a1) of the process of the present invention and added and the reaction mixture was stirred at 30 °C for 20 h according to step b) or b1) of the process of the present invention.

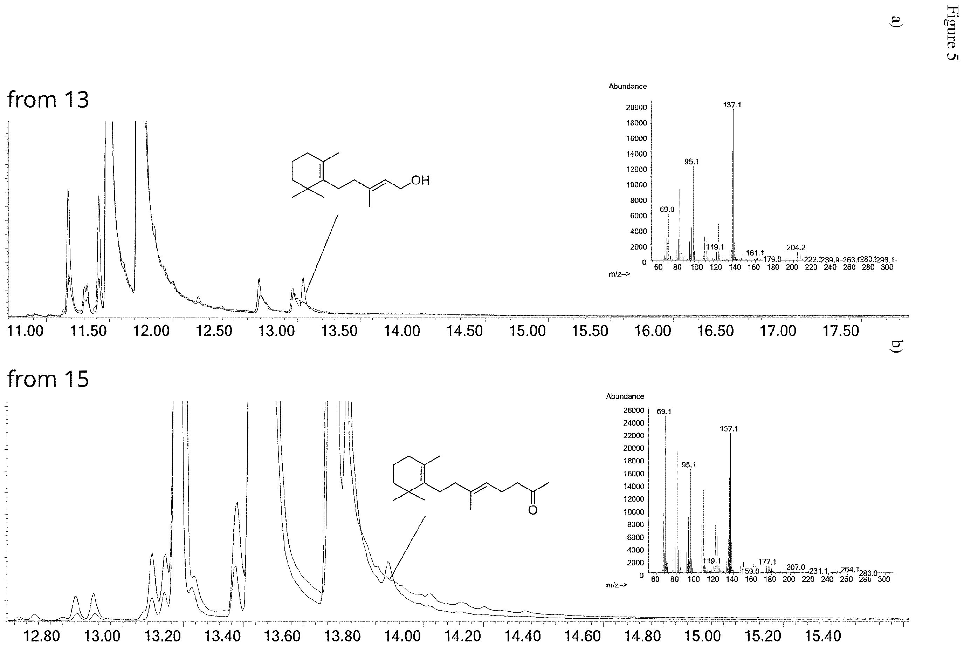

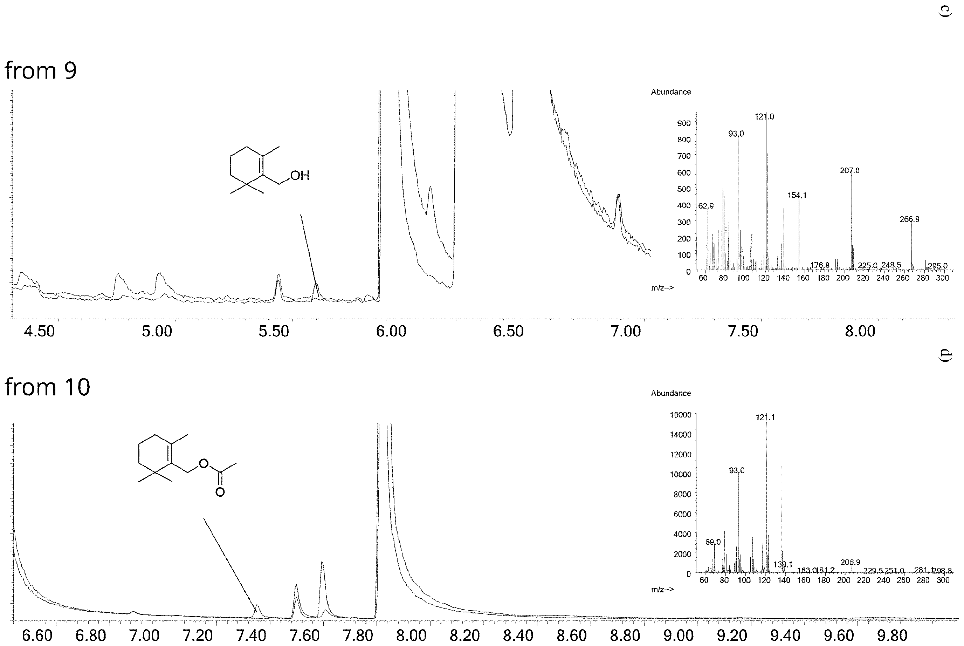

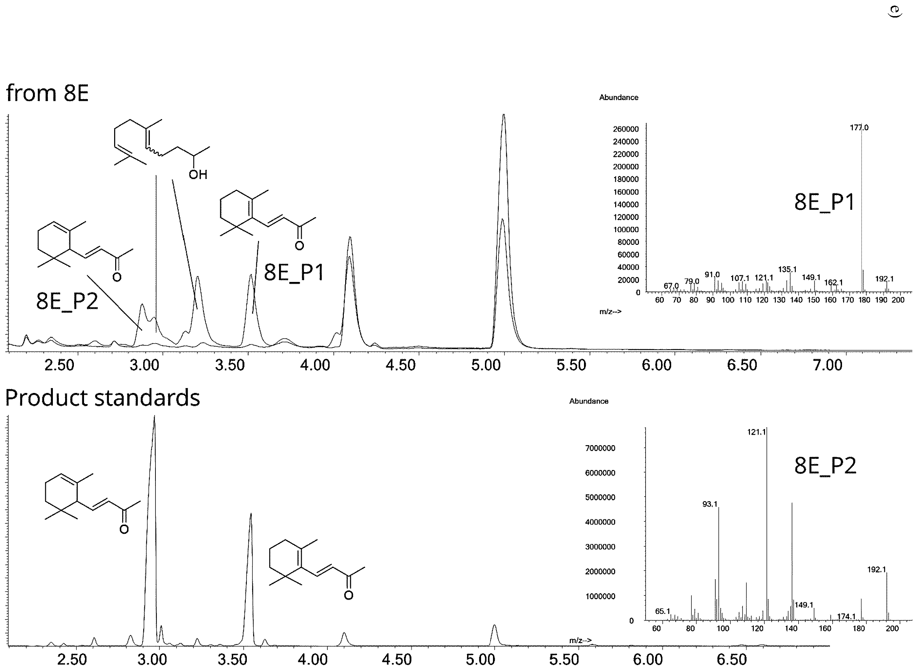

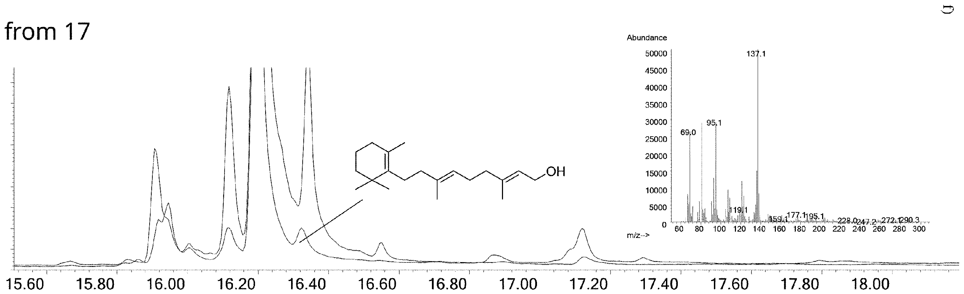

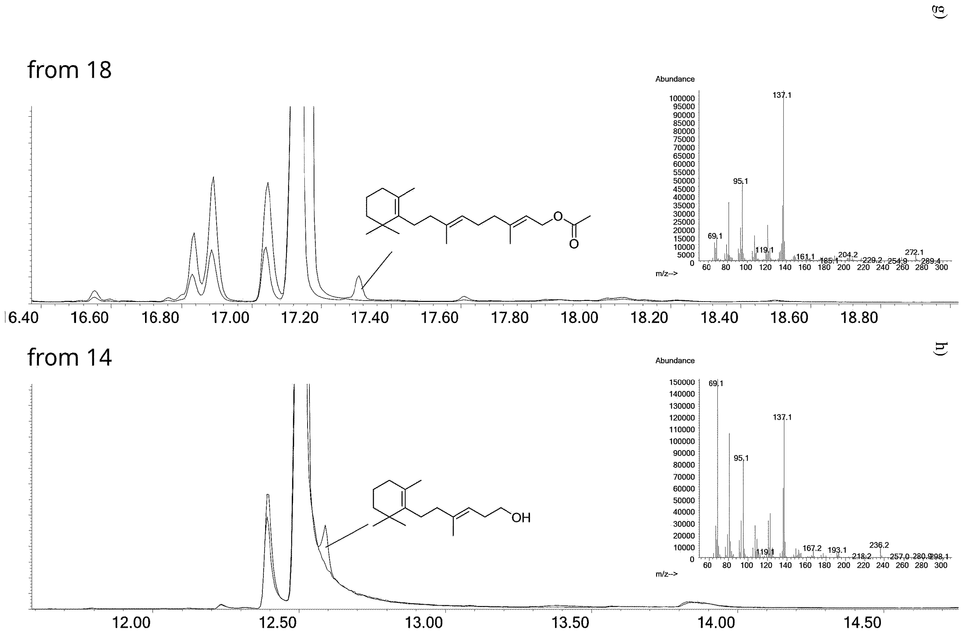

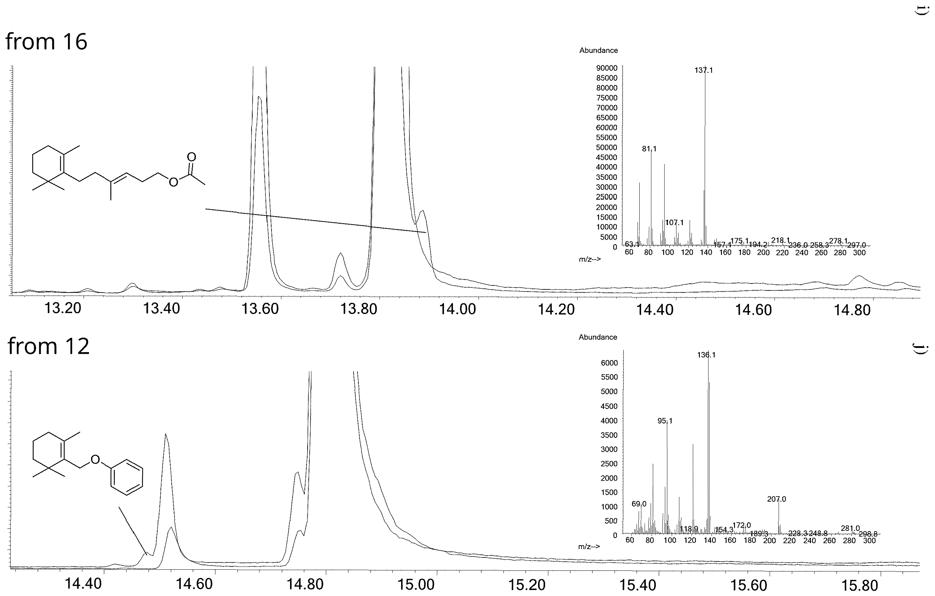

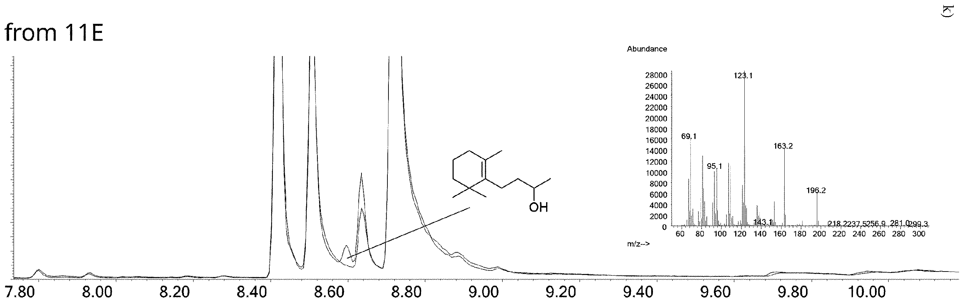

Surprisingly, 13 more substrates were converted, which was confirmed with empty vector and buffer controls. As shown in the scheme 2 below linear precursor geraniol corresponding to 9 and geranyl acetate corresponding to 10 were cyclized to the corresponding monocycles with 0.2 and 2.5%, respectively. E-geranyl acetone corresponding to 11 and E-pseudoionone corresponding to 8were cyclized to the corresponding monocycles with 1.2, 31% (β-deprotonation) and 13% (α-deprotonation) conversion rates. The non-natural terpene geranyl phenyl ether corresponding to 12 was cyclized to the corresponding monocycle with 0.1% conversion rate. Farnesol corresponding to 13, homofarnesol corresponding to 14 and farnesylacetone corresponding to 15 were cyclized to the corresponding monocycles with 1, 6 and 1%, respectively. Homofarnesyl acetate corresponding to 16, geranyl geraniol corresponding to 17 and geranyl geranyl acetate corresponding to 18 were cyclized to the corresponding monocycles with conversion rates of 3, 5 and 3%, respectively. In figure 5 a) to j) the respective GC chromatograms including the respective mass spectra as inlet of compounds of formula II from their respective substrates of formula I with molecule numbers 8E (figure 5 e), 9 (figure 5 c), 10 (figure 5 d), 11 (figure 5k), 12 (figure 5 j), 13 (figure 5 a), 14 (figure 5 h), 15 (figure 5 b), 16 (figure 5 i), 17 (figure 5 f), 18 (figure 5 g) are shown converted using LcyB_C as biocatalyst.

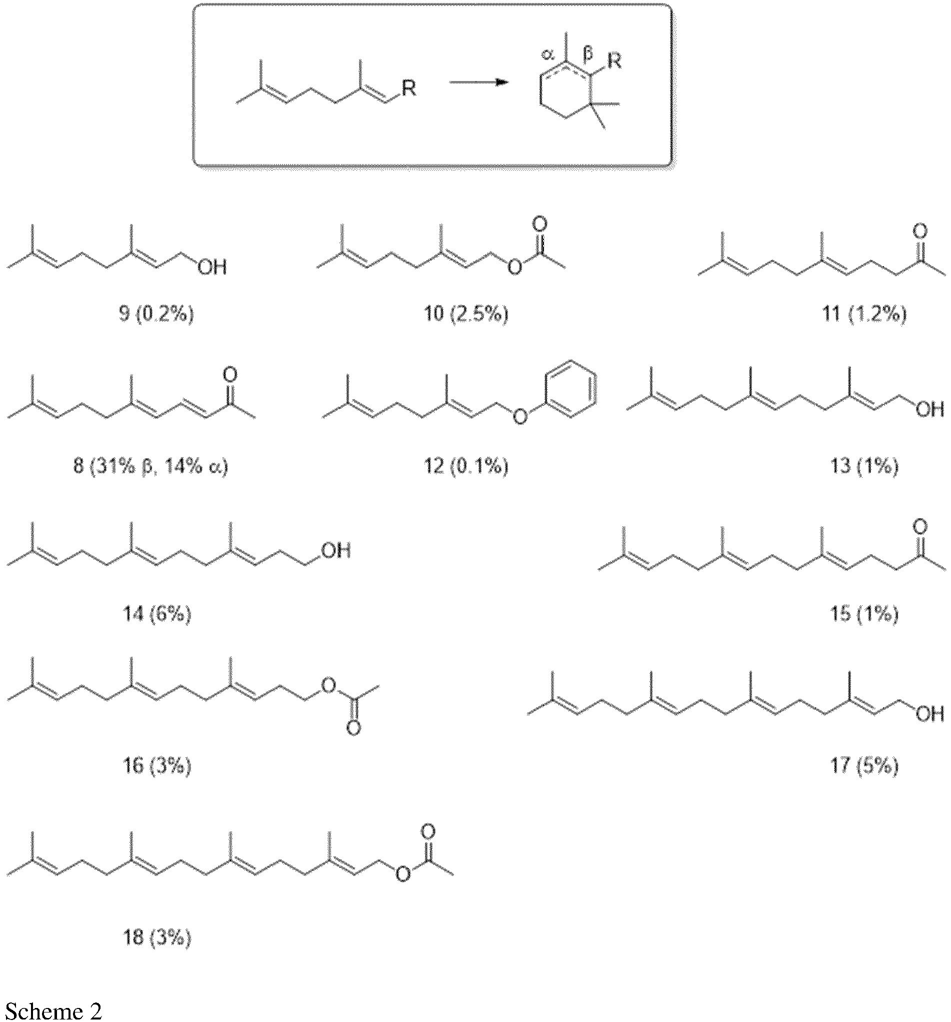

### Sequence alignment of Leys (SEQ ID Nos 1 to 9)

Multiple-sequence alignment was performed with Clustal Omega, F. Sievers, A. Wilm, D. Dineen, T. J. Gibson, K. Karplus, W. Li, R. Lopez, H. McWilliam, M. Remmert, J. Söding, J. D. Thompson, D. G. Higgins, Mol. Syst. Biol. 2011, 7, using the amino acid sequences according to SEQ ID Nos 1 to 9, wherein CrtY-Pan corresponds to SEQ ID No. 6, LcyE-Dsa corresponds to SEQ ID No. 5, Lcy-Zma corresponds to SEQ ID No. 4, LcyE-Arath_q38932 corresponds to SEQ ID No. 3, Lcy-Csi corresponds to SEQ ID No. 8, Crtl-syn corresponds to SEQ ID No. 7. LcyB-Arath_q38933 corresponds to SEQ ID No. 2, LcyB-CAPAN_q43415 corresponds to SEQ ID No. 1, Tomato-B corresponds to SEQ ID No. 9. This program allows the simultaneous alignment of multiple sequences, highlighting conserved amino acids (*) very similar (:), similar sites (.) and differing sites ( ). The result of this alignment is presented in form of a matrix below:

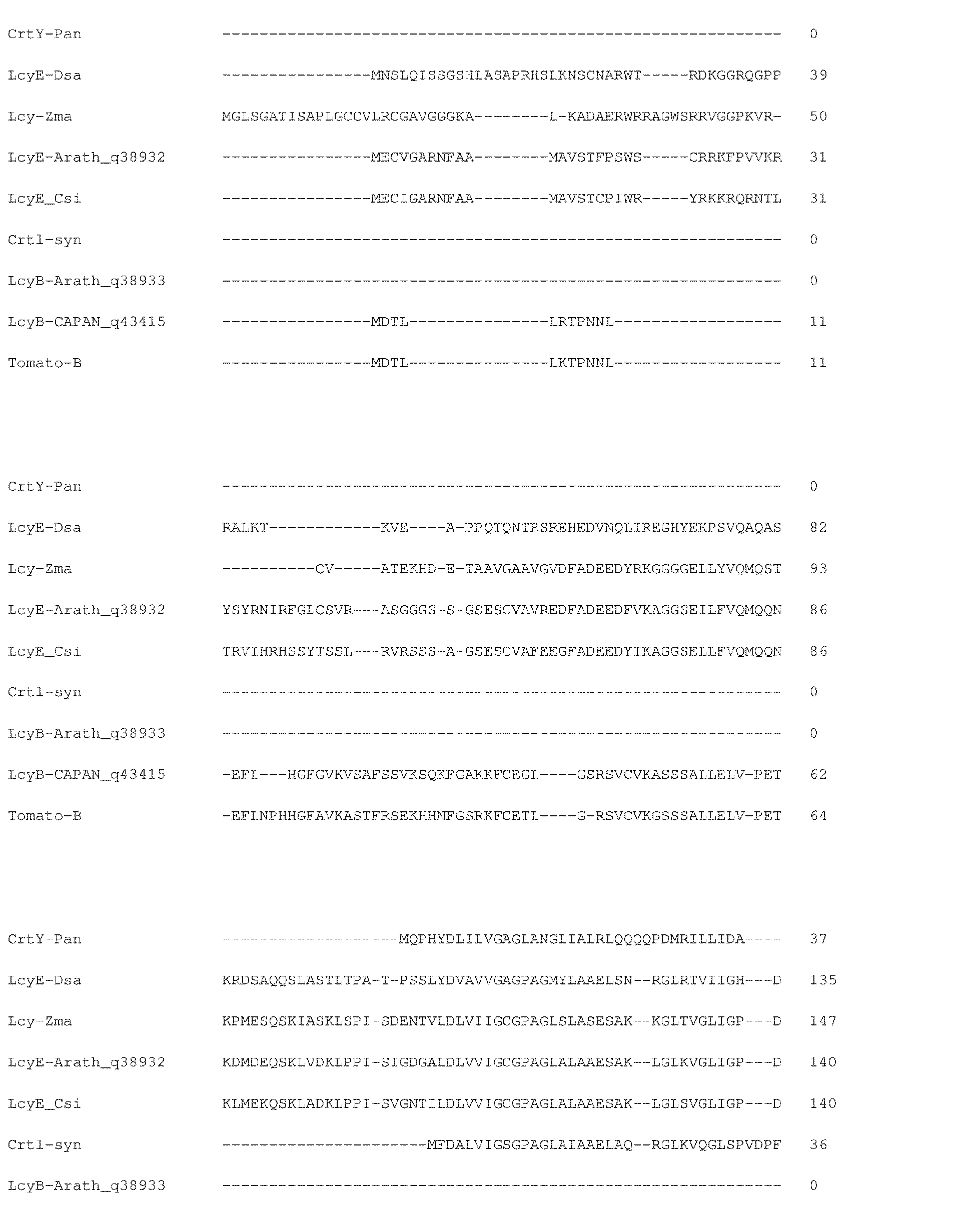

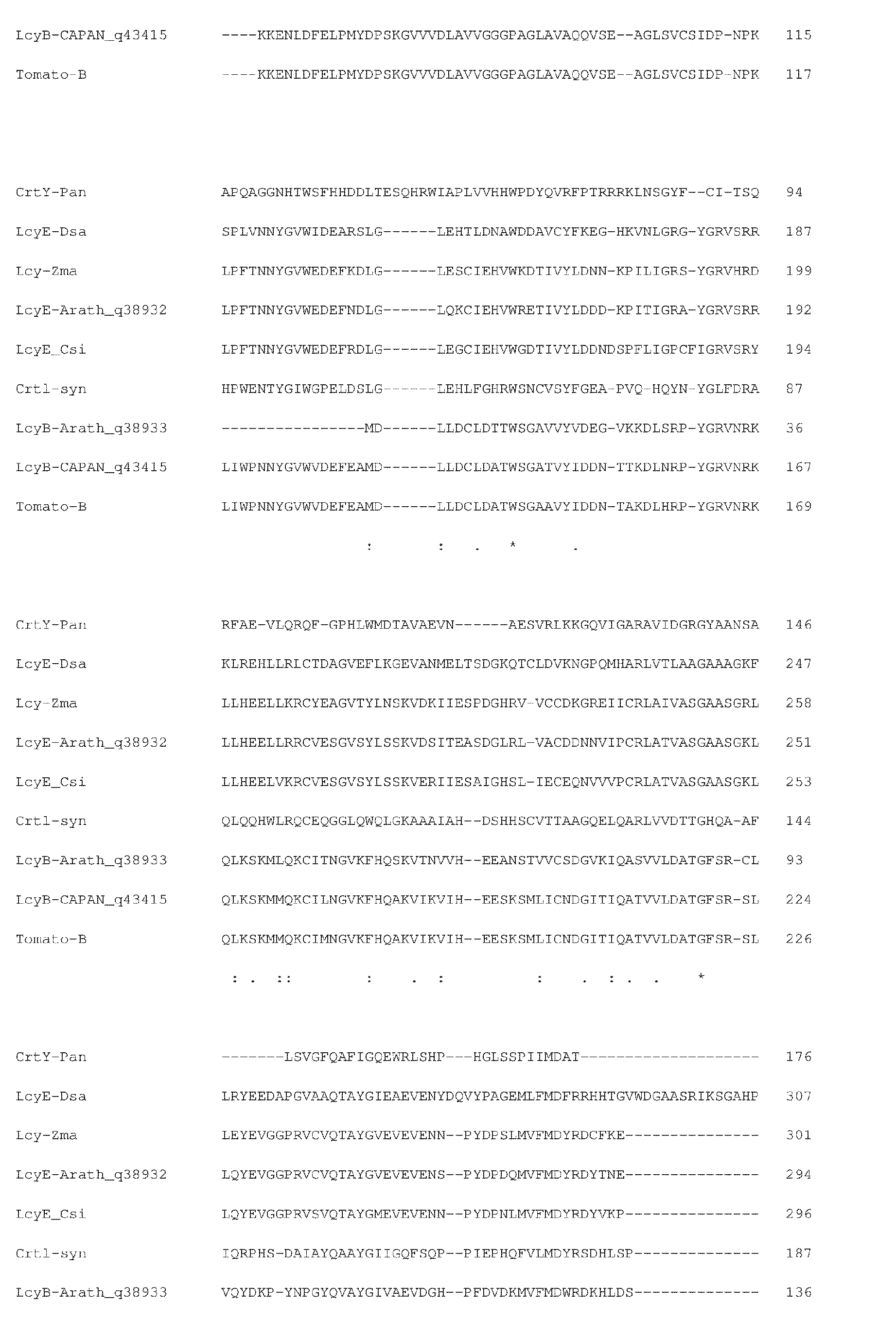

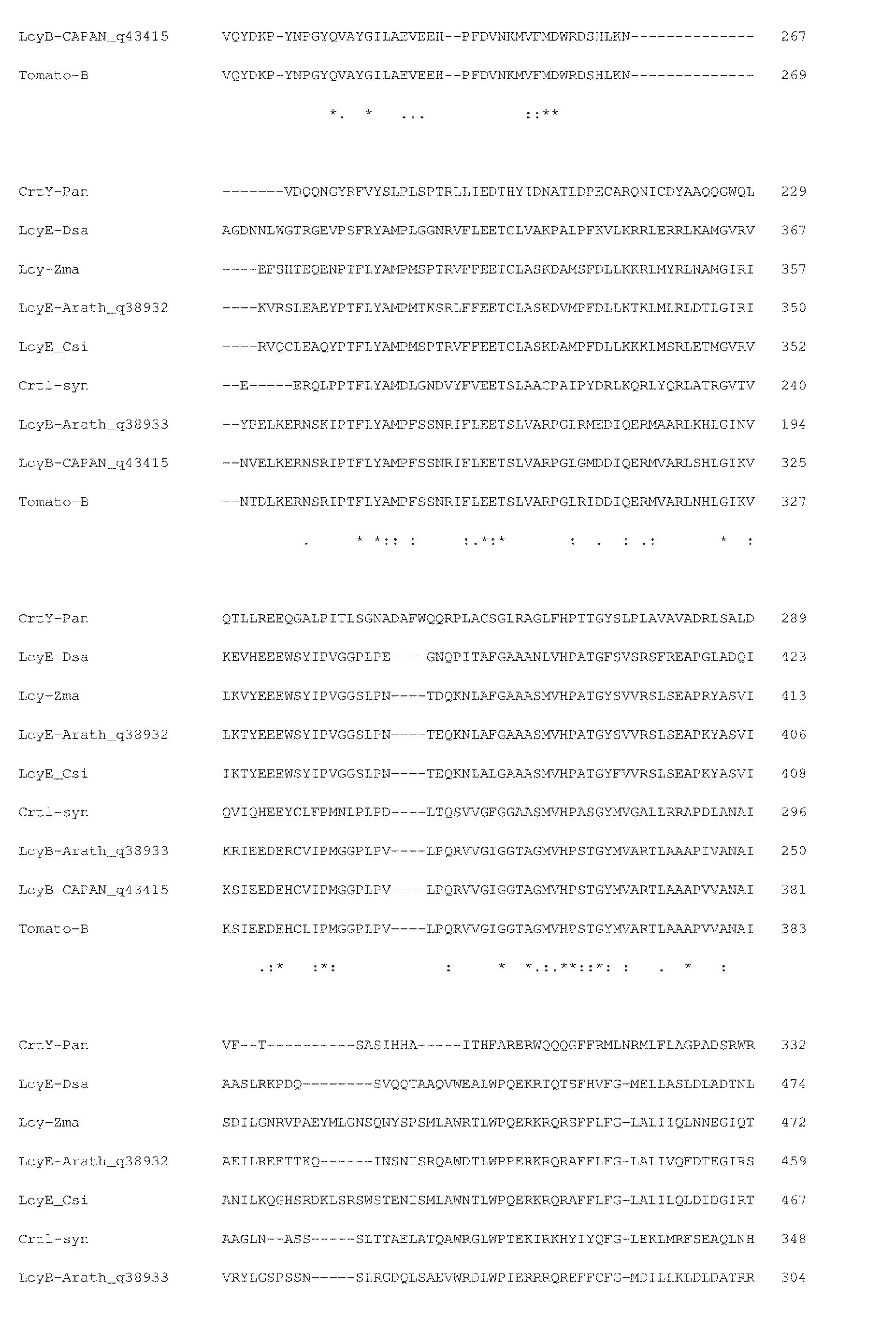

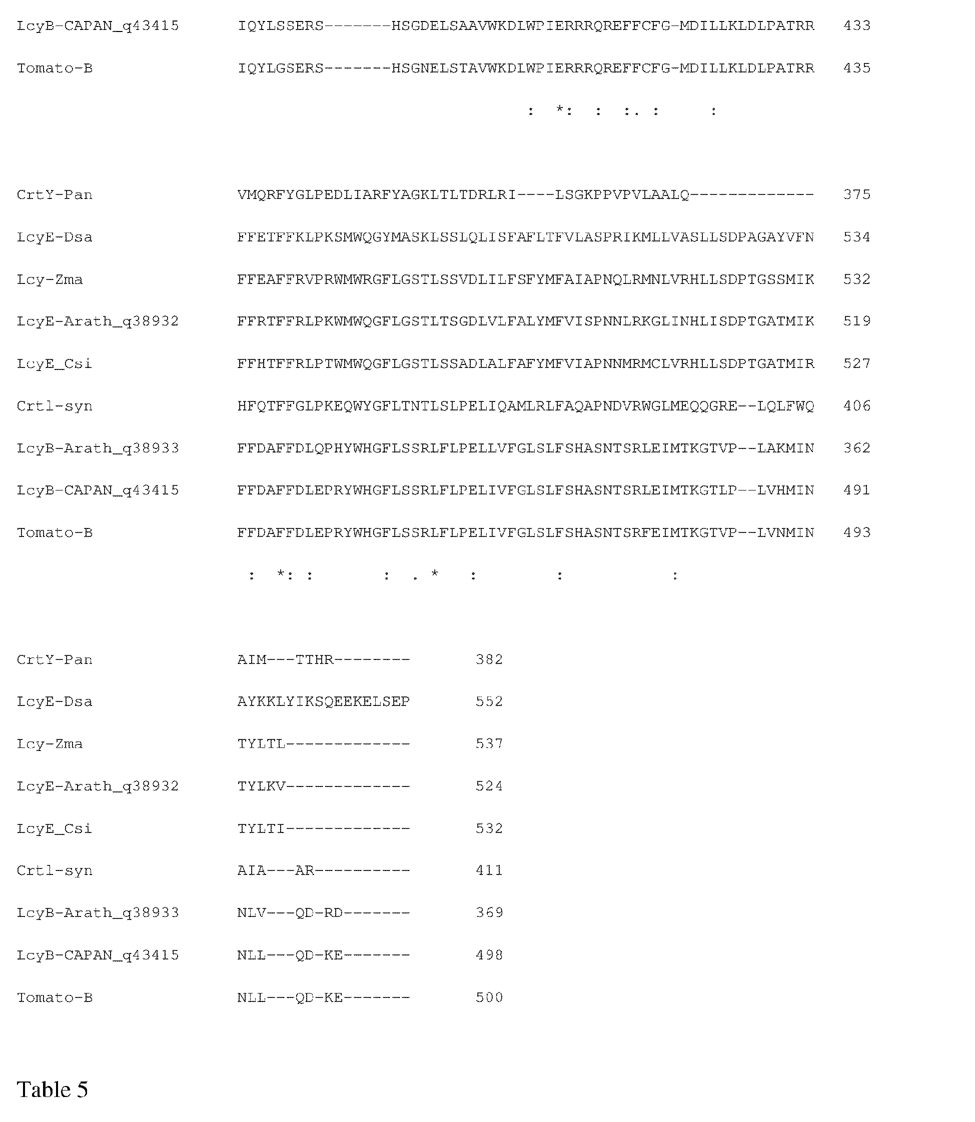

The following multiple-sequence alignment was also performed with Clustal Omega using the amino acid sequences according to SEQ ID Nos 1, 2, 7 and 9, wherein Crtl-syn corresponds to SEQ ID No 7, LcyB-Arath_q38933 corresponds to SEQ ID No 2, LcyB-CAPAN_q43415 corresponds to SEQ ID No 1, Tomato-B corresponds to SEQ ID No 9. This program allows the simultaneous alignment of multiple sequences, highlighting conserved amino acids (*) very similar (:), similar sites (.) and differing sites ( ). The result of this alignment is presented in form of a matrix below:

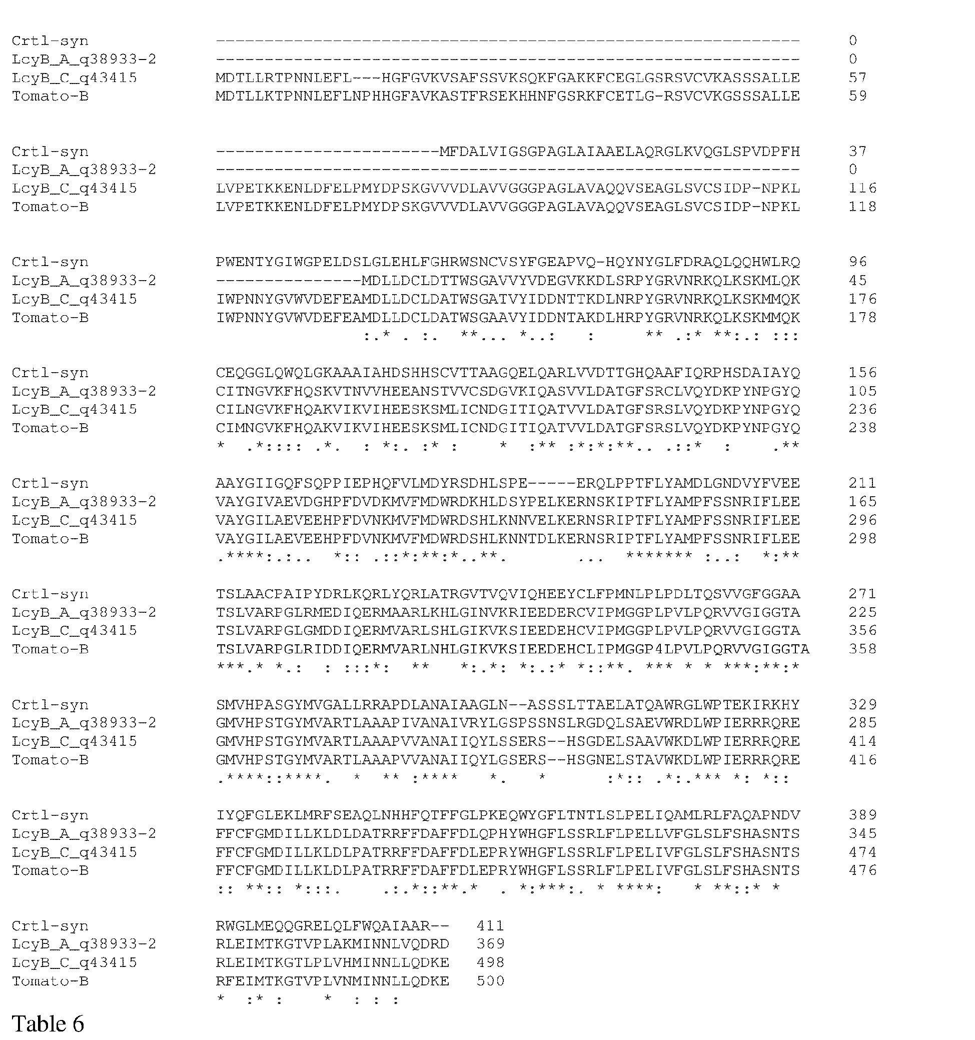

In the matrices of table 5 and table 6 the various corresponding amino acid sequences are presented in rows which are aligned so that identical amino acids at corresponding positions are arranged in columns marked by * and wherein gaps in between identical and thus conserved amino acids are indicated by "-".

## Claims

1. A process for preparing a composition comprising a compound of formula II 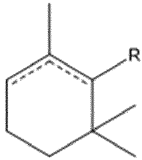 with R having the meaning of R in formula I, comprising the following steps:
a) providing a substrate of formula I 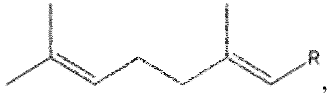 wherein R is an alkyl residue with at most 20 carbon atoms or an alkyl residue with at most 20 carbon atoms including at least one functional group, in particular wherein the functional group comprises at least one halide, O, N, S, P and/or unsaturated C atom,
at least one reaction medium and at least one biocatalyst comprising a protein with the enzymatic activity of a small target (ST)-lycopene-β-cyclase (LcyB), which ST-LcyB comprises an amino acid sequence of a LcyB and is **characterised by** the presence of a first set of conserved amino acids F281, Y283, E295, T297, S298, E332, P337, G353, A356, G364 and M366 by reference to the wild type LcyB of SEQ ID No. 1,
b) mixing and reacting the substrate with the reaction medium and the biocatalyst provided in step a) and under reaction conditions so as to convert the substrate of formula I to the compound of formula II and
c) obtaining a composition comprising the compound of formula II.

2. The process according to claim 1, wherein the functional group is selected from the group consisting of a fluoride group, a chloride group, a bromide group, an iodide group, a hydroxy group, a ketone group, an aldehyde group, a halo formyl group, a carbonate ester group, a carboxylate group, a carboxyl group, a carboalkoxy group, an ether group, an acetal group, a carboxylic anhydride group, a carboxamide group, an amidine group, an amine group, a ketimine group, an imide group, an azide group, a diimide group, a cyanate group, an isocyanate group, a nitrate group, a nitrile group, a isonitrile group, a nitrite group, a nitro group, a nitroso group, a oxime group, a pyridyl group, a carbamate group, a sulfhydryl group, a sulfide group, a disulfide group, a sulfinyl group, a sulfonyl group, a sulfino group, a sulfo group, a thiocyanate group, a isothiocyanate group, a carbonothioyl group, a carbothioic S-acid group, a carbothioic O-acid group, a thioester group, a thionoester group, a carbodithioic acid group, a carbodithio group, a phosphino group, a phosphono group, a phosphate group, an alkenyl group, in particular an unsaturated methylene group, an alkynyl group, in particular a methine group, and a combination thereof.

3. The process according to claim 1 or 2, wherein the at least one functional group is selected from the group consisting of a hydroxy group, a ketone group, a carbonate ester group, an oxybenzyl group and a combination thereof.

4. The process according to any one of the preceding claims, wherein R is selected from a group consisting of CH₃, 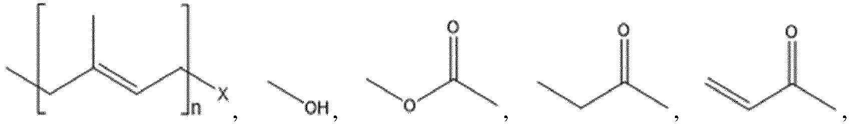 and 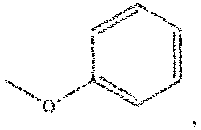 wherein X is selected from a group consisting of H, OH, 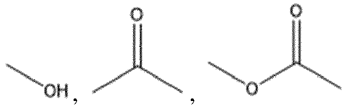 and 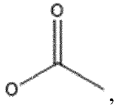 and
wherein n is 1 to 3.

5. The process according to any one of the preceding claims, wherein the substrate is selected from the group consisting of the substrates having the substrate number 8, 9, 10, 11, 12, 13 ,14, 15, 16, 17 and 18

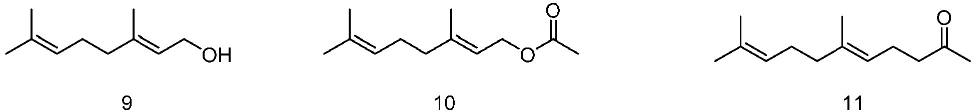

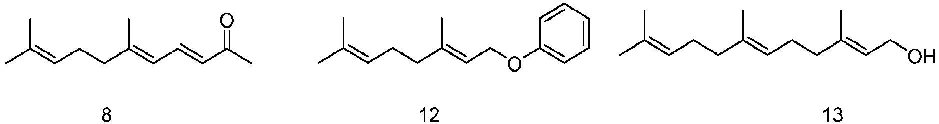

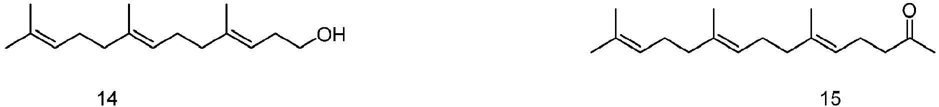

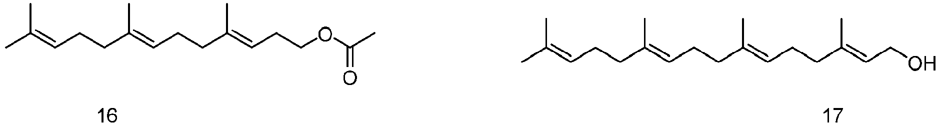

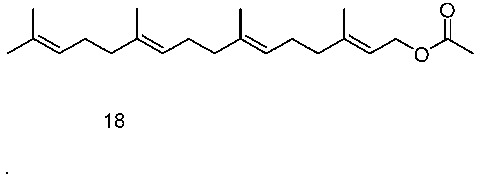

6. The process according to any one of the preceding claims, wherein the reaction medium comprises a buffer, in particular selected from the group consisting of phosphate buffer, in particular potassium phosphate buffer, in particular 10× phosphate buffer, a potassium buffer, in particular MES/potassium buffer, in particular MES/KOH buffer, TRIS-Maleate buffer, citrate buffer and acetate buffer, in particular citrate buffer.

7. The process according to any one of the preceding one of the preceding claims, wherein the reaction medium comprises at least one co-factor, in particular NADPH or FMN.

8. The process according to any one of the preceding claims, wherein the reaction temperature in step c) is from 20 to 50 °C, in particular 25 to 35 °C, preferably 30 °C.

9. The process according to any one of the preceding claims, wherein the pH of the reaction medium is from 5 to 10, in particular 6.

10. The process according to any one of the preceding claims, wherein the biocatalyst is an isolated protein, a protein mixture, or a protein-comprising system, which system is a host cell, a host cell lysate, a freeze-dried host cell, a host cell extract or a host cell fraction and wherein the protein has the enzymatic activity of a lycopene β-cyclase (LcyB).

11. The process according to any one of the preceding claims, wherein the biocatalyst is a carrier-supported biocatalyst.

12. The process according to any one of the preceding claims, wherein the ST-LcyB is **characterised by** the presence of a second set of conserved amino acids L134, Y148, G162, R166, Q168, L169, C177, G181, K188, H194, S199, G206, Q210, A211, V214, D216, T218, Q226, Y235, A238, Y239, 1241, P249, V256, R261, H264, L265, P279, T280, L282, A284, M285, F293, E296, S298, L299, A301, P303, R313, R317, L318, V325, 1328, C334, M338, P341, L342, P343, L345, Q347, V350, V351, G354, M358, V359, Y365, M366, V367, L371, P375, A378, N379, A380, 1381, L385, S390, L396, W400, L404, W405, P406, R411, F418, G419, L423, F435, L441 W446, G448, F449, L450, L456, P457, E458, L459, L464, L466, F467, A470, N472, R475, M479 and L486 by reference to the wild type LcyB of SEQ ID No. 1.

13. The process according to any one of the preceding claims, wherein the ST-LcyB is **characterised by** the presence of a third set of conserved amino acids W142, G219, Q236, G240, M258, D259, F281, Y283, E295, T297, G322, E332, P337, G353, A356, H360, P361, G364, A374, E408, F438 and L454 by reference to the wild type LcyB of SEQ ID No. 1.

14. The process according to any one of the preceding claims, wherein the ST-LcyB is **characterised by** the presence of an amino acid sequence of a wild type LcyB of SEQ ID Nos 1, 2, 7 or 9.

15. The process according to any one of the preceding claims, wherein in step d) the compound of formula II is isolated from the composition obtained in step c).

16. A composition obtainable according to any one of the processes according to any one of claims 1 to 15.
